# EUROPEAN PATENT APPLICATION

(11) **EP 4 656 202 A1**
(43) Date of publication of application: **03.12.2025**
(21) Application number: 23920421.7
(22) Date of filing: 09.02.2023
(51) Int. Cl.: A61K 38/20, A61K 31/573, A61P 17/00, A61P 37/00

(54) **TREATMENT OF ATOPIC DERMATITIS WITH POLYETHYLENE GLYCOL-MODIFIED INTERLEUKIN 2, GLUCOCORTICOID AND HYALURONIC ACID**

(71) Applicant: Xie, Yanhui, Shanghai 201100 (CN)
(72) Inventor: ZHU, Yuanling, Lichuan City Enshi, Hubei 445400 (CN); XIE, Yanhui, Shanghai 201100 (CN)
(74) Representative: Turner, Craig Robert
(86) International application number: PCT/CN2023/075121
(87) International publication number: WO 2024/164213

(57) **Abstract**

Disclosed in the present invention is a pharmaceutical composition for treating atopic dermatitis, the pharmaceutical composition comprising polyethylene glycol-modified interleukin 2, a glucocorticoid and low-molecular-weight hyaluronic acid, and optionally comprising a pharmaceutically acceptable carrier; and further provided is 10KD PEG-modified IL2 and the use thereof in treating type I allergic diseases.

## Description

### Technical Field

The present invention relates to a polyethylene glycol-modified interleukin 2 (IL-2/IL2), a glucocorticoid and a hyaluronic acid for treating Type I hypersensitivity (allergy) reaction, in particular, for treating atopic dermatitis.

### Background Art

IL-2, as a multi-directional cytokine, prodominantly promotes the proliferation Treg cells at low concentrations, but at high concentrations, prodominantly promotes the proliferation of Teff cells, so it is difficult to control the dose when it is used directly in immune modulation therapy and the efficacy is unstable [GRAβHOFFH, et al. Low-Dose IL-2 Therapy in Autoimmune and Rheumatic Diseases. Front Immunol, 2021 Apr 1; 12: 648408]. In the previous study, it was found that using PEG modified IL-2 combined glucocorticoids can effectively promote the proliferation of Treg cells without significant effects on Teff [WU K, et al. Short-term intratracheal use of PEG-modified IL-2 and glucocorticoid persistently alleviates asthma in a mouse model. Sci Rep, 2016 Aug. 16; 6: 31562; WU Min et al., "Combination of dexamethasone with IL-2 selectively induces the expansion of CD4+ CD25+ FOXP3+ regulatory T cells in vivo and suppresses graft versus host disease", Chin J Hematol, 2009, 30(11): 726-30; MA J, et al. Alleviating allergic airway diseases by means of short-term administration of IL-2 and dexamethasone. J Allergy Clin Immunol, 2011, 127(6): 1447-56 e6], assumed that because the spatial conformation of the PEG-modified IL-2 is changed, weakening the affinity of the IL-2 receptor β subunit, resulting in a large decrease in the proliferation effect of CD4+CD25-T cells with a small effect on the proliferation effect of CD4+CD25+Treg, and finally leading to the prodominant proliferation of Treg cells. At the same time, the low affinity PEG-IL-2 can still promote the phosphorylation of FoxO3a in Treg cells with the help of CD25 molecules, enhance the resistance to glucocorticoids to induce apoptosis of immune cells, and have no protective effect on CD4+CD25-T cells.

Regulatory T cells (Tregs) are important immunosentinels, and negatively regulate the immunization by secreting anti-inflammatory cytokines (such as IL-10, TGF-β), highly expressing PD-1 and CTLA- 4, releasing granular enzymes and perforin to induce apoptosis of effector T cells (Teff), so as to maintain immune balance homeostasis of the body and avoid excessive immune over-activation. Immune imbalance resulted from effector T cell proliferation or Treg cell reduction or functional deficiency is an important link for a variety of allergic diseases and autoimmune diseases, including allergic asthma, allergic dermatitis, allergic gastroenteritis, systemic lupus erythematosus, polyendocrine disease X linkage syndrome and the like [DENG G, et al. Foxp3 Post-translational Modifications and Treg Suppressive Activity. Front Immunol, 2019 Oct. 18, 10:2486; RAFFIN C, et al. T(reg) cell-based therapies: challenges and perspectives. Nat Rev Immunol, 2020, 20(3): 158-72; SHEVYREV D, TERESHCHENKO V. Treg Heterogeneity, Function, and Homeostasis. Front Immunol, 2020 Jan 14; 10:3100; ATTIAS M, et al. Mechanisms of human FoxP3(+) Treg cell development and function in health and disease. Clin Exp Immunol, 2019, 197(1): 36-51]. Such diseases can be effectively treated with regulatory T cells.

Skin is the biggest barrier tissue of the body, and long-term contact with various stimuli from external environments, so the proportion of Treg cells in normal skin is higher than that in peripheral blood to maintain local immune homeostasis [ALI N, ROSENBLUM MD. Regulatory T cells in skin. Immunology, 2017, 152(3): 372-81; SCHARSCHMIDT TC, et al. A Wave of Regulatory T Cells into Neonatal Skin Mediates Tolerance to Commensal Microbes. Immunity, 2015, 43(5): 1011-21]. However, in the inflammatory skin with damaged barrier, local immunity is over-activated, the effector cells and cytokines in the pro-inflammatory direction are greatly increased, and the compensatory increase of Treg cells is insufficient to maintain the immune balance [BILLROTH-MACLURG A C, et al. Regulatory T Cell Numbers in Inflamed Skin Are Controlled by Local Inflammatory Cues That Upregulate CD25 and Facilitate Antigen-Driven Local Proliferation. J Immunol, 2016, 197(6): 2208-18].

Type I hypersensitive (allergy) reactions have the following characteristics: 1) high prevalence, affecting 20-40% of human population; 2) fatality, such as anaphylactic shock and asthma attack; 3) delayed unhealing, recurrence, embarrassment, such as allergic rhinitis, atopic dermatitis (eczema); 4) Type I allergy diseases are a class of diseases with the same pathophysiology, including three phases: allergen-priming (sensitization) phase, allergen-challenge phase, and effect phase, involving allergens, allergins (allergen specific IgE), CD4+Th2 helper cells, IL-4, IL-13, IL-5 cytokines, mast cells, and basophils. Type I allergy diseases includes: 1) systemic anaphylaxis including drug anaphylactic shock and serum anaphylactic shock; 2) respiratory allergies such as allergic rhinitis and allergic asthma; 3) gastrointestinal allergic reactions such as allergic gastroenteritis; 4) skin allergic reactions such as urticaria, atopic dermatitis (eczema) and angioedema.

Atopic dermatitis (AD) is a skin manifestation of Type I allergies. AD affects 10%-20% of the population worldwide whose prevalence is rising year by year as the level of environmental hygiene increases in life. The chronic relapse of AD has a serious impact on long-term quality of life of patients. At present, clinical medication aim to relieve symptoms such as itching, swelling and exudation in the acute phase of dermatitis, and achieve certain therapeutic effects, but the efficacy of drugs is limited and the duration is short, and the side effects of long-term medication are also not ignored. Therefore, there is a need in the art, which is safe, effective and long-lasting action, for treatment and prevention of atopic dermatitis.

### Brief summary of the invention

In the first aspect, there is provided a pharmaceutical composition for the treatment of atopic dermatitis, comprising a polyethylene glycol-modified interleukin 2 (PEG-IL2), a glucocorticoid and a small molecular weight hyaluronic acid (HA), and optionally a pharmaceutically acceptable carrier.

In the second aspect, there is provided a method for treating atopic dermatitis in a subject, comprising administering to the subject a therapeutically effective amount of a PEG-IL2, a glucocorticoid and a small molecular weight HA.

In the third aspect, there is provided use of a PEG-IL2, a glucocorticoid and HA in the preparation of a medicament for the treatment of atopic dermatitis, and a PEG-IL2, a glucocorticoid and a HA for use in the treatment of atopic dermatitis, wherein the HA comprises a small molecular weight HA.

In the fourth aspect, there is provided a kit comprising a PEG-IL2, a glucocorticoid and a HA, wherein the HA comprises a large molecular weight HA and/or a small molecular weight HA.

In the fifth aspect, there is provided use of a HA in the preparation of an agent for increasing the efficacy of a PEG-IL2 and/or a glucocorticoid in treating atopic dermatitis, and a HA for use in increasing the efficacy of a PEG-IL2 and/or a glucocorticoid in treating atopic dermatitis, wherein the HA comprises a small molecular weight HA.

In the sixth aspect, there is provided use of a 10KD PEG modified IL2 in the preparation of a medicament for the treatment of type I allergies.

In the seventh aspect, there is provided a method for treating an I-type allergy in a subject, comprising administering to the subject a 10 KD PEG modified IL2.

In the eighth aspect, a 10 KD PEG modified IL2 is provided.

### Brief Description of the Figures

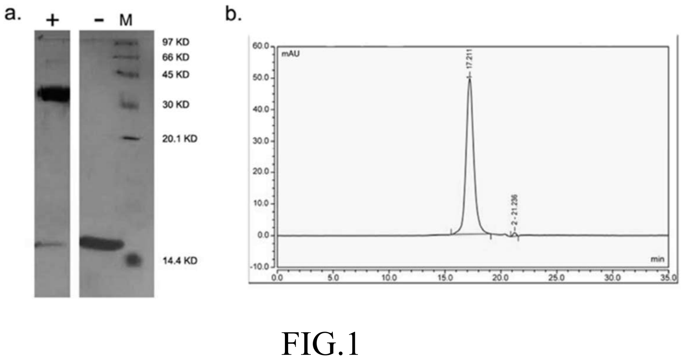

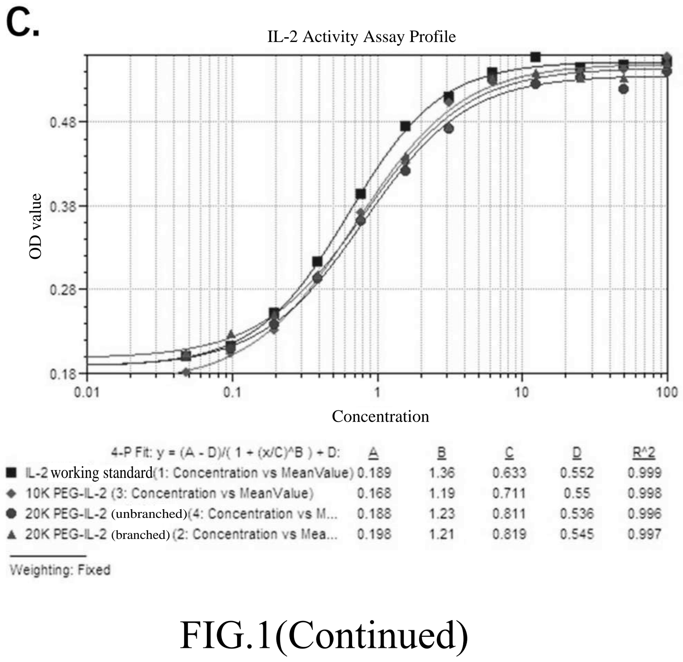

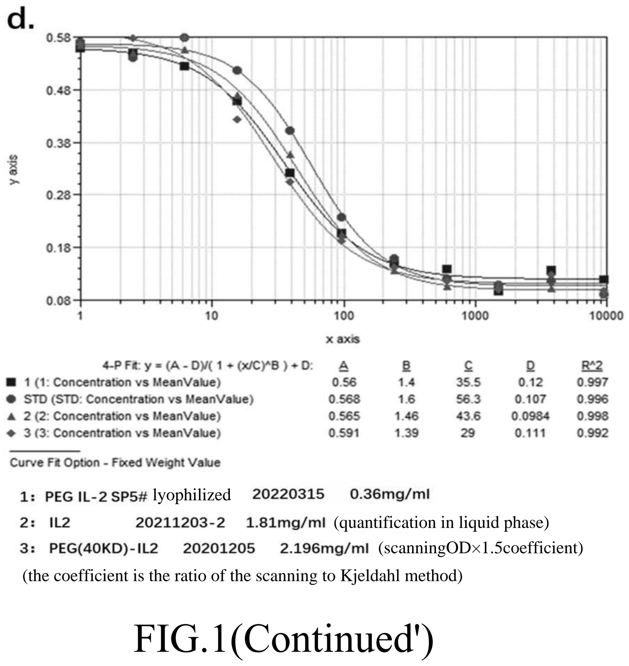

FIG. 1: Preparation of a polyethylene glycol (PEG) modified interleukin 2. (a) The recombinant human interleukin 2 before (-) and after (+) modified with a polyethylene glycol (PEG) were conducted to electropherensis with SDS-PAGE and then silver-stained. (b) Monitoring of a PEG modified IL-2 after purification. M, marker. (c) Activity assay of a recombinant human interleukin 2, recombinant human interleukin 2 modified with polyethylene glycol (PEG) of different molecular weights and structures. (d) Activity assay of a recombinant human interleukin 2 modified with a polyethylene glycol (PEG) (40kD-PEG-IL-2) and a recombinant human interleukin 2 (IL- 2).

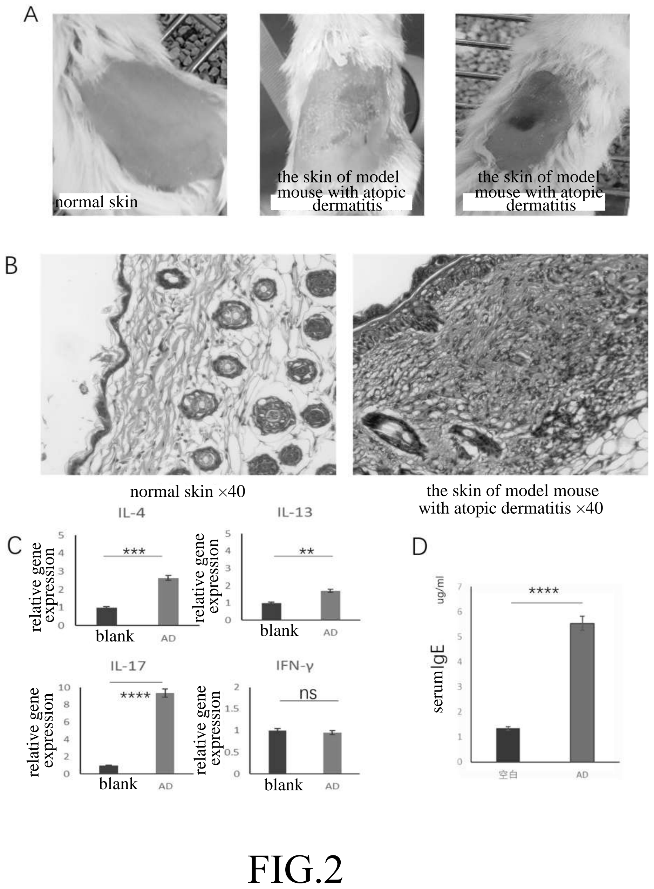

FIG. 2: OVA induced atopic dermatitis (AD) mouse model. A is the skin photos of normal and dermatitis mice, AD skin redness, scaling, lesion, etc. can be observed; B is two groups of skin pathological H&E staining images, x40 times, it can be seen that AD skin was significantly thicker, and a large amount of inflammatory cells infiltrated; C is the mRNA expression profile of skin inflammatory factors via quantitative RT-PCR, the coordinate is the 2^(-ΔΔCT) value of qPCR mRNA of experimental mice/physiological mice, the blank group was control "1", and the expressions of IL-4, IL-13 and IL-17 in AD skin were obviously upregulated wherein the difference was statistically significant, and IFN-γ expression had no statistical difference; D is the serum IgE concentration detected by ELISA, and the serum IgE in AD mice was significantly increased, with a statistical difference. In the figure, ** represents P < 0.01, *** represents P < 0.001, **** represents P = 0.00, and ns representes P > 0.05.

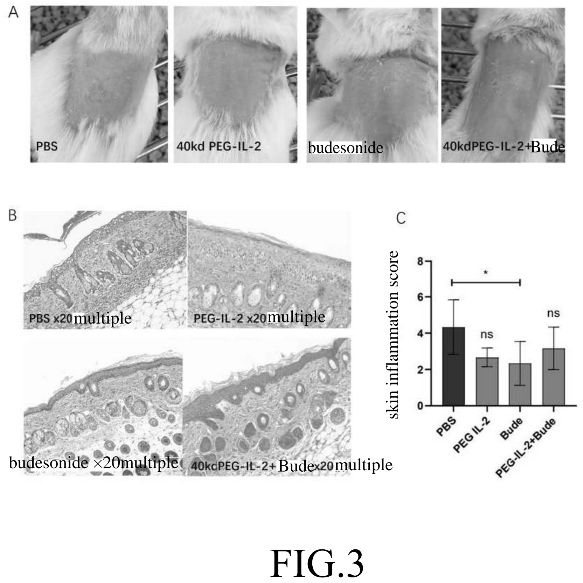

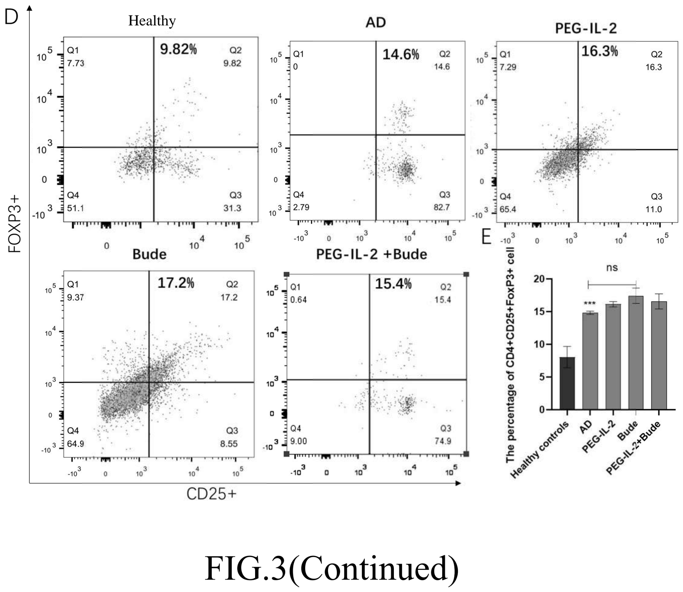

FIG. 3: A is a photograph of skin 3 days after treating mice with the buffer (PBS), 40kD PEG-IL-2, budesonide (Bude), 40kD PEG-IL2+budesonide, and the dermatitis of each group of mice were relieved; B is a skin pathological H&E staining image of each group, and the infiltration degree of inflammatory cells of skin was similar, a multiple of x20; C is a statistical diagram of dermatitis scores of mice in each group, only the PBS group had a statistical difference with the group treated with budesonide alone; D is the proportions of skin Tregs in the blank control group, the AD model group and each treatment group in flow cytometry; E is a statistical graph of Treg proportion, compared with the blank group, each group had a statistical difference, but there was no statistical difference among the treatment groups. In the figure, * represents P<0.05, *** represents P<0.001, and ns represents P > 0.05.

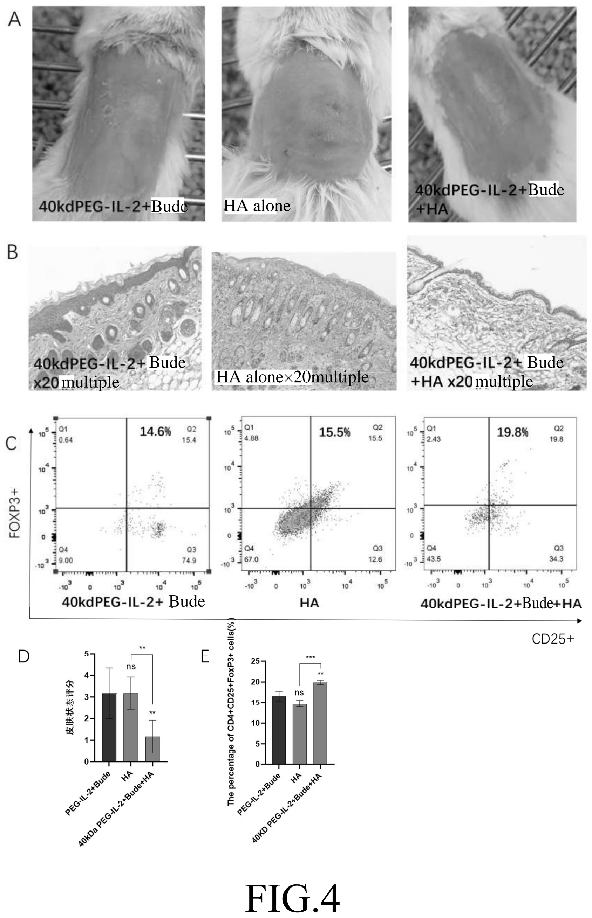

FIG. 4: A is skin photos of dermatitis mice administered with 40kdPEG-IL-2 + budesonide (Bude), HA alone and 40kdPEG-IL-2+budesonide+HA, respectively, and the improvement of skin damage in the last group was significantly better than the first two groups; B is skin pathological H&E staining images corresponding to each groups, the 40kdPEG-IL-2+budesonide+HA group had a skin inflammatory cell infiltration degree significantly lower than the other two groups, the multiple x20; C is the ratio of CD25⁺FOXP3⁺ Treg cells in skin CD4⁺ cells of each group by flow cytometry; D is a dermatitis score statistical chart, 40kdPEG-IL-2+budesonide plus HA group, compared to the group without HA and the group treated with HA alone, had a reduced dermatitis score, and the difference was statistically significant; E is a skin Treg statistical graph, and the difference between the HA group and the group without HA or the group treated with HA alone was statistically significant. In the figure, ** represents P < 0.01, *** represents P < 0.001, and ns represents P > 0.05.

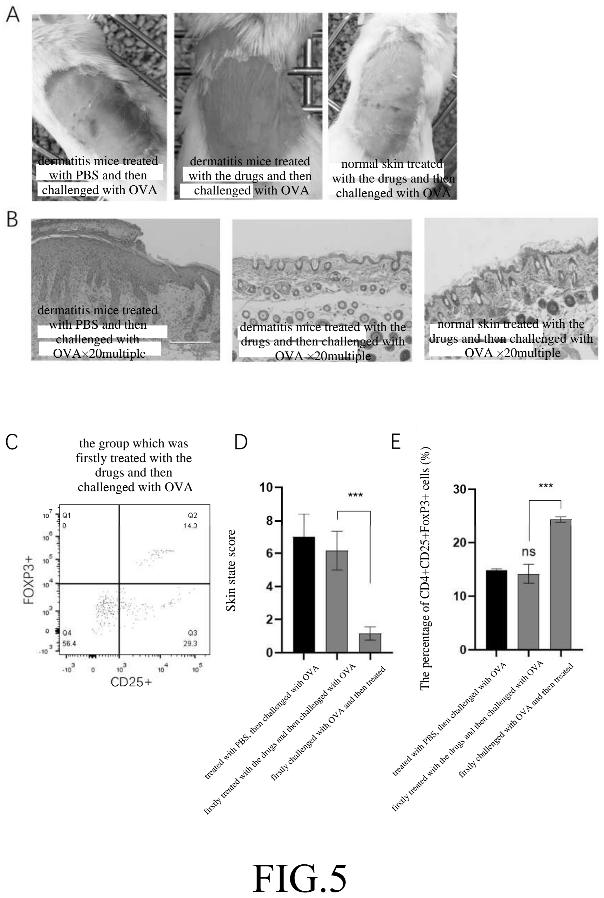

FIG. 5: A is skin photos of the group of dermatitis mice treated with PBS, the group in which mice dermatitis was firstly induced and then treated (HA+PEG-IL-2+budesonide) and then contacted OVA, and the goup in which the normal skin state of the allergen-sensitized mice were first administrated for 3 days and then induced via contacting OVA, and the pictures showed that the administration was for only three days in the dermatitis state, which could prevent the occurrence of dermatitis that was challenge by the antigen OVA for seven consecutive days; B is skin pathological H&E staining images of respective groups, multiple ×20, showing that the infiltration of inflammatory cells in the skin in the group administered after dermatitis was obviously reduced; C is the proportion of CD25+FOXP3+Treg cells in skin CD4+cells when the normal skin of allergen-sensitized mice was administered and then contacted OVA for 3 days; D is the score statistical chart of dermatitis, and the two groups administered with drugs had a statistical significance; E is the statistical chart of skin Treg, and the difference between the two groups administered with drugs was statistically significant. In the figure, *** represents P < 0.001, ns represents P > 0.05.

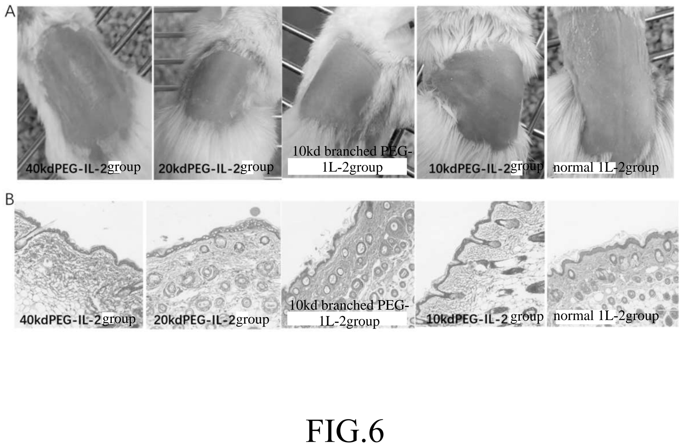

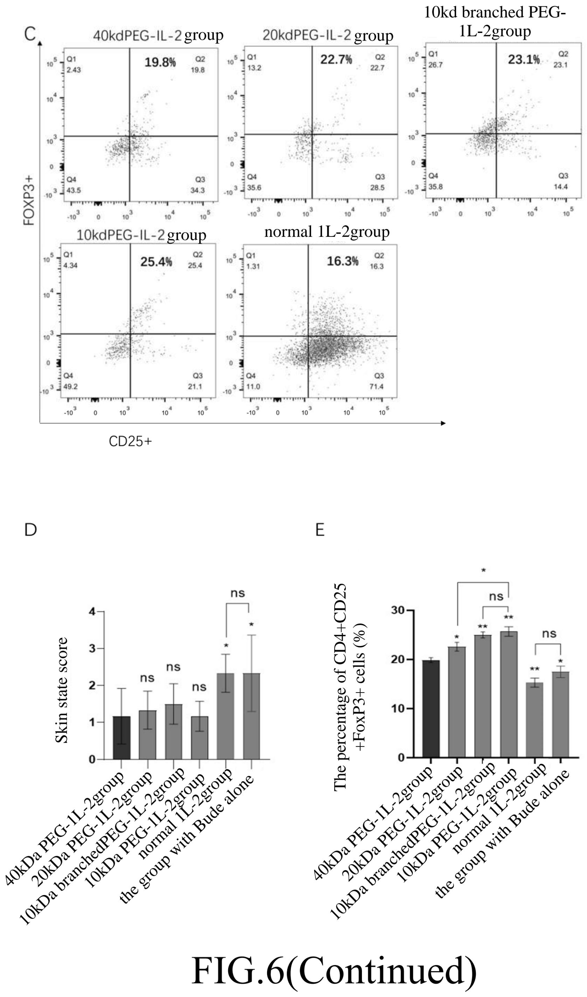

FIG. 6: In the case that budesonide+HA is unchanged, after treatment in combination with 40kD PEG-IL-2, 20kD PEG-IL-2, 10kD branched PEG-IL-2, 10kD unbranched PEG-IL-2, and normal IL-2, A is skin pictures of mice in each group; B is respective skin pathological H&E staining images of each group, multiple x20; C is the proportion of Treg in skin of each group via flow cytometry; D is the score statistical chart of dermatitis in each group, and compared to the 40kD-PEG-IL-2 group, no statistical difference was observed in the 20kD-PEG-IL-2 group, the 10kD-PEG-IL-2 group (branched), and 10kD-PEG-IL-2 group (non-branched), and there is no statistical difference between the normal IL-2 group and the group with budesonide alone, and these two groups respectively had a statistically difference compared to the 40kD-PEG-IL-2 group; E is skin Treg statistical chart, compared between the groups, combination with the 10kd PEG-IL-2 maximally upregulated Treg, and the difference has statistical significance. The ratio of Tregs in the group combined with normal IL-2 was the lowest, and there was no difference from the group with budesonide alone. In the figure, * represents P < 0.05, ** represents P < 0.01, *** represents P < 0.001, ns represents P > 0.05.

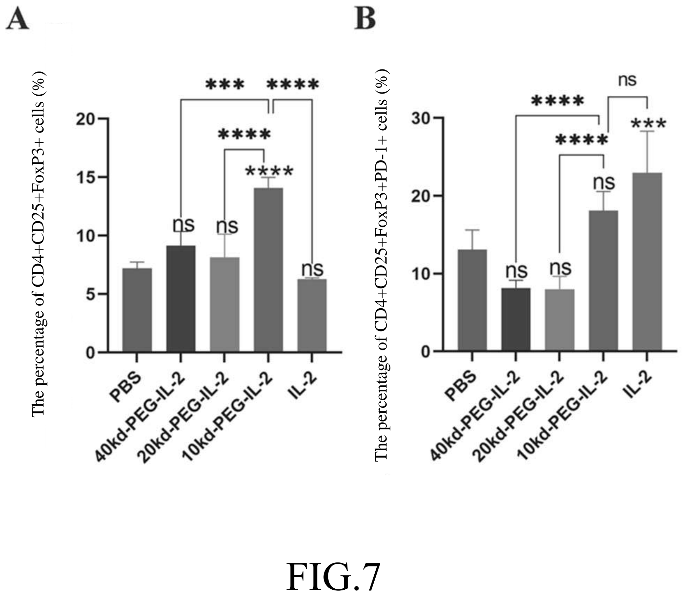

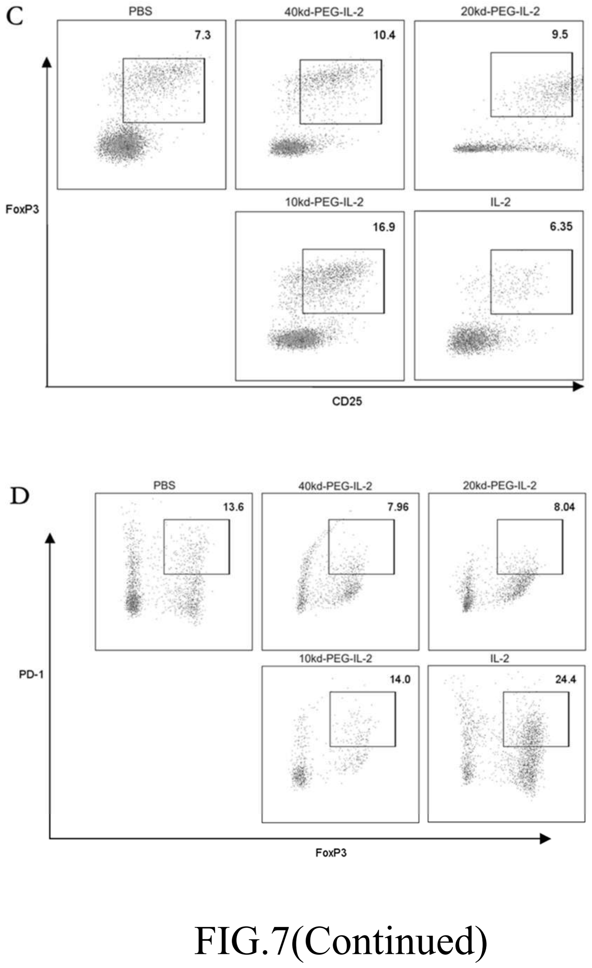

FIG. 7: Percentage of FoxP3+ cells and PD-1+ cells in CD3+CD4+CD25+ cells after 3 days of treatment by intraperitoneal injection of PEG-IL-2 modified by non-branched PEG of different molecular weights in physiological model mice. The percentages of CD25+FoxP3+ cells in spleen CD3+CD4+ lymphocytes and of PD-1+ cells in CD3+CD4+CD25+FoxP3+ cells were analyzed. FIG. A is the percentage of CD4+CD25+FoxP3+ cells and FIG. B is the percentage of CD4+FoxP3+PD-1+ cells. FIG. C is a flow cytometry diagram of CD4+CD25+FoxP3+ cells. FIG. D is a flow cytometry diagram of CD4+FoxP3+PD-1+ cells. This study showed that only 10kD-PEG-IL2 can significantly up-regulate regulatory T cells (Tregs) in the physiological mouse model and induce such Treg cell to highly express PD-1 (PD-1 can enhance the inhibitory function of Treg cells). In the figure, *** represents P < 0.001, **** represents P < 0.0001.

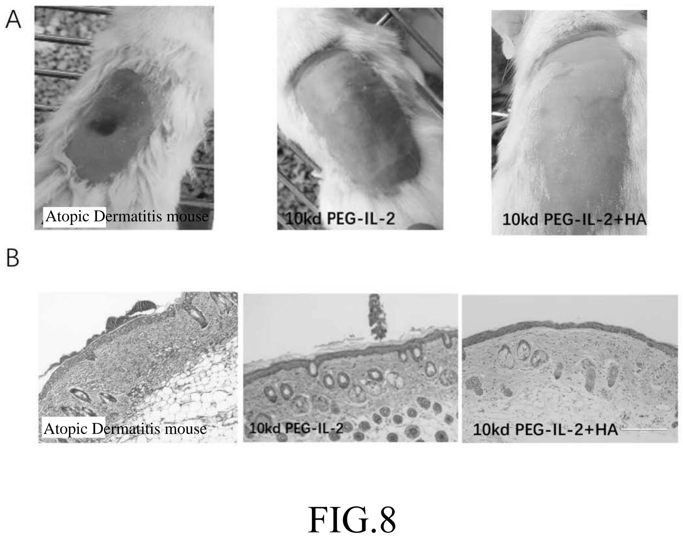

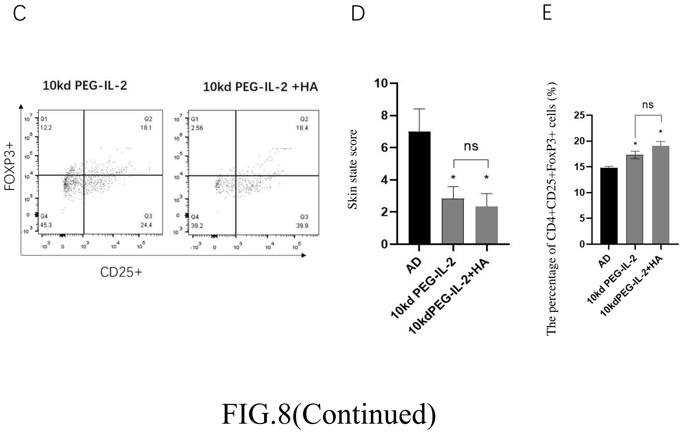

FIG. 8: A is skin photographs of a dermatitis mouse and mice after treatment with 10kdPEG-IL-2 with or without HA, respectively; B is skin pathological H&E staining images of respective groups, multiple x20; C is the ratio of CD25+FOXP3+ Treg cells in skin CD4+ cells detected by flow cytometry after treating mice with 10kdPEG-IL-2 combined with or without HA; D is a dermatitis score statistical chart, showing two treatment groups had statistically significant differences compared with the control group, but there was no statistical difference between the two treatment groups; E is a skin Treg statistical chart, showing the two treatment groups had statistically significant differences compared with the control group, but there was no statistical difference between the two treatment groups. In the figure, * represents P < 0.05, ns represents P > 0.05.

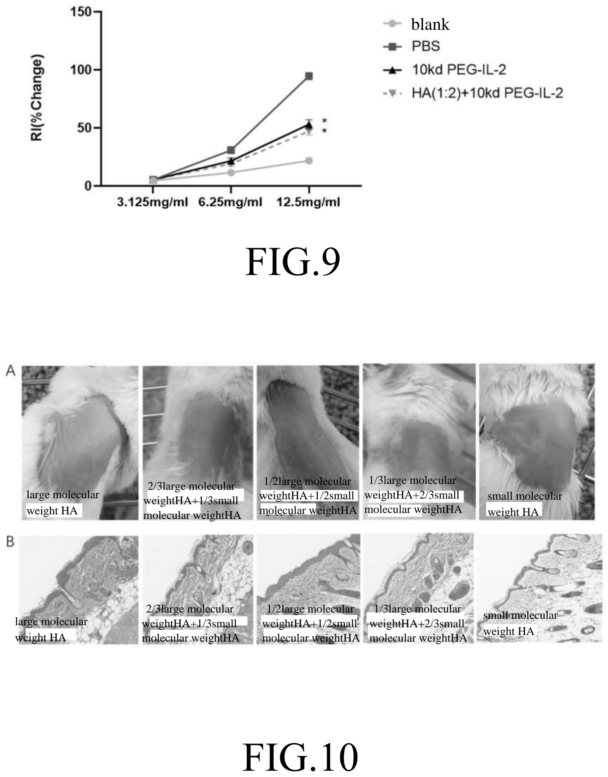

FIG. 9: AHR analysis of an allergic asthma mouse model with drugs and PBS, and healthy mice, and changes in lung (airway) resistance (RI), which was used as an indicator for evaluating airway reactivity. * indicates p < 0.05 compared with mice in the blank control group and the PBS treated group. Local use of 10kD-PEG-IL2 significantly reduced airway resistance.

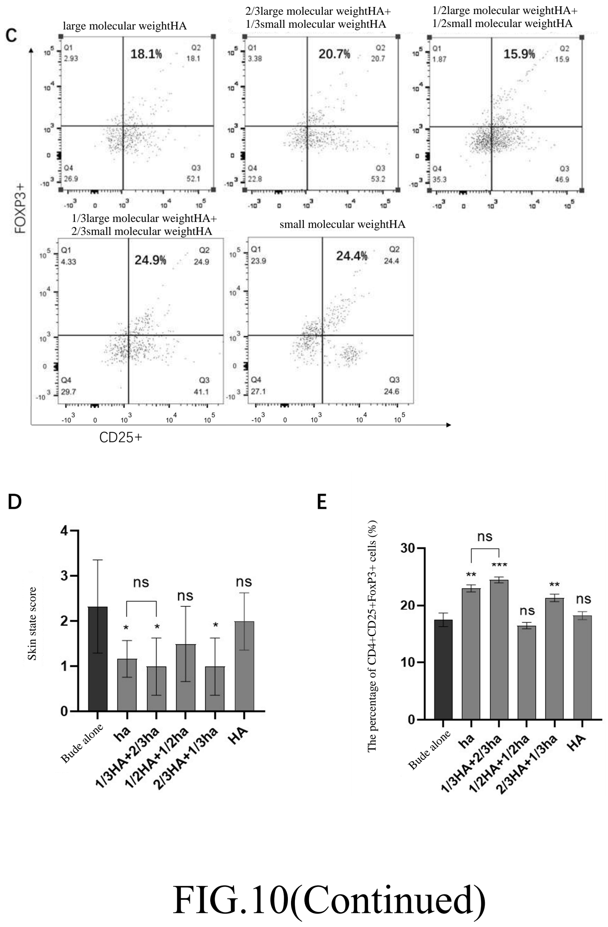

FIG. 10: A is skin photographs when 10kd PEG-IL-2+budesonide was fixed, respectively in combination with the large molecular weight HA, 2/3 large molecular weight HA+1/3 small molecular weight HA, 1/2 large molecular weight HA+1/2 small molecular weight HA, 1/3 large molecular weight HA+2/3 small molecular weight HA, and small molecular weight HA; B is respective skin pathological H&E staining images, multiple x20; C is the proportion of CD25+FOXP3+ Treg cells in skin CD4+ cells detected via flow cytometry; D is the skin dermatitis score statistical chart, and comparing the HA (HA) groups with the group with budesonide (Bude) alone, in the 1/3 large molecular weight HA+2/3 small molecular weight HA group and the 2/3 large molecular weight HA+1/3 small molecular weight HA group, dermatitis improvement were most obvious, small molecular weight HA group was second, there were statistical differences, and in the large molecular weight HA group and 1/2 large molecular weight HA+1/2 small molecular weight HA group, dermatitis improvement had no statistical difference; E is the skin Treg statistical graph, compared with the group with budesonide alone, the combination of 1/3 large molecular weight HA+2/3 small molecular weight HA maximally upregulated Treg, and the statistical significance was maximal, but there was no statistical significant difference compared to the small molecular weight HA group (P > 0.05). ns represents P > 0.05, * represents P < 0.05, ** represents P < 0.01, *** represents P < 0.001.

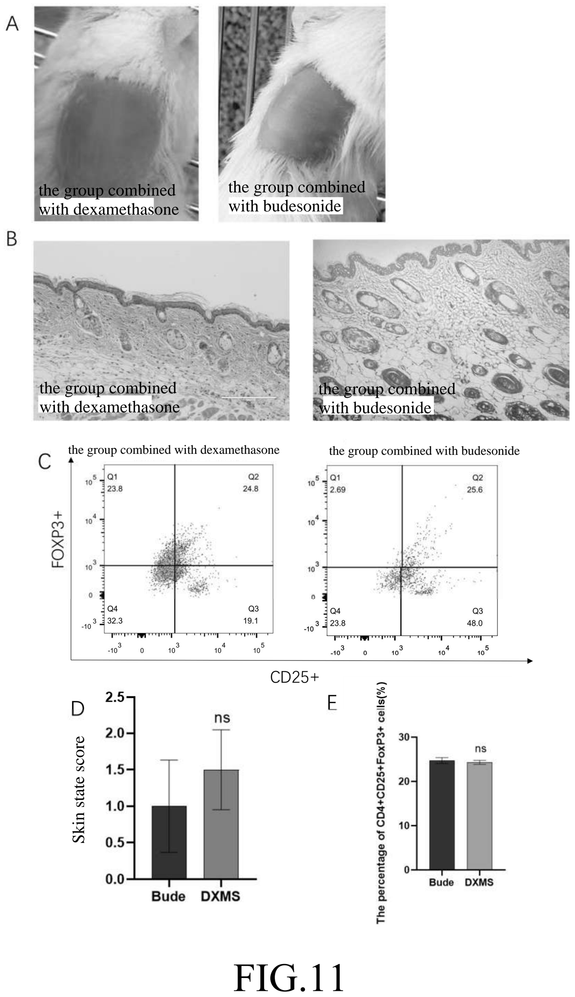

FIG. 11: A is skin manifestation after 3 days of administration of 10kDa PEG-IL-2 + dexamethasone (DXMS) or budesonide (Bude)+HA (large molecular weight: small molecular weight is 1:2); B is the skin pathological H&E staining images, multiple X20; C is the proportion of CD25+FOXP3+ Treg cells in skin CD4+ cells detected by flow cytometry; D is the dermatitis score statistical chart, and there was no statistical difference between the group combined with dexamethasone and the group combined with budesonide (P > 0.05); E is the percentage of skin Treg, and there was no statistical difference between the group combined with dexamethasone and the group combined with budesonide (P > 0.05).

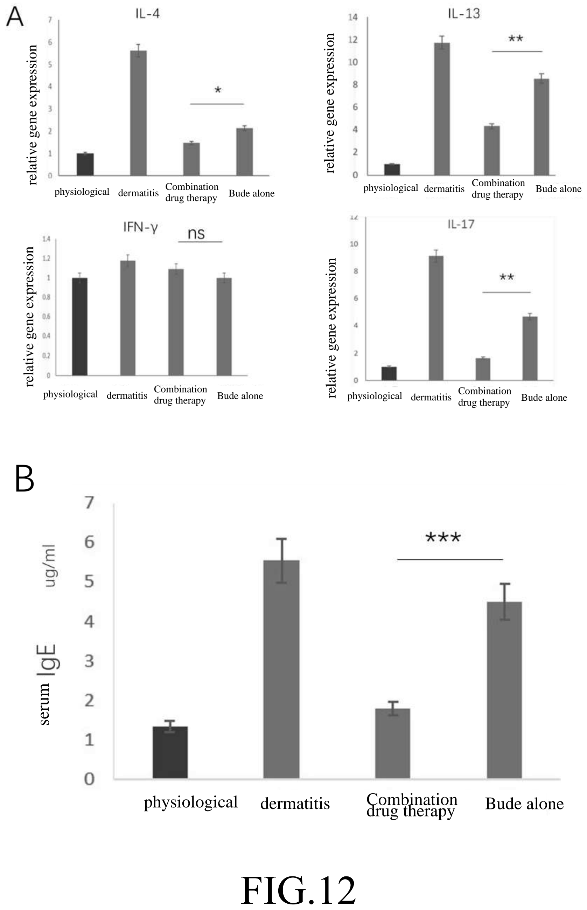

FIG. 12: A is the expressions of IL-4, IL-13, IL-17 and IFN-γ in mice skin in the blank control, dermatitis model, the optimal combination group determined by the foregoing experiments, and budesonide (glucocorticoid, the clinical first-line therapy for atopic dermatitis) treated group, in the combination groups IL-4, IL-13 and IL-17 were down-regulated more than the budesonide group, and the difference had statistical significance, while the expression of IFN-γ in each group had no difference (P > 0.05); B is the serum IgE concentration of each group detected by ELISA, the combination group reduced serum IgE more than the budesonide group, and the difference had statistical significance; ns represents P > 0.05, * represents P < 0.05, ** represents P < 0.01, *** represents P < 0.001.

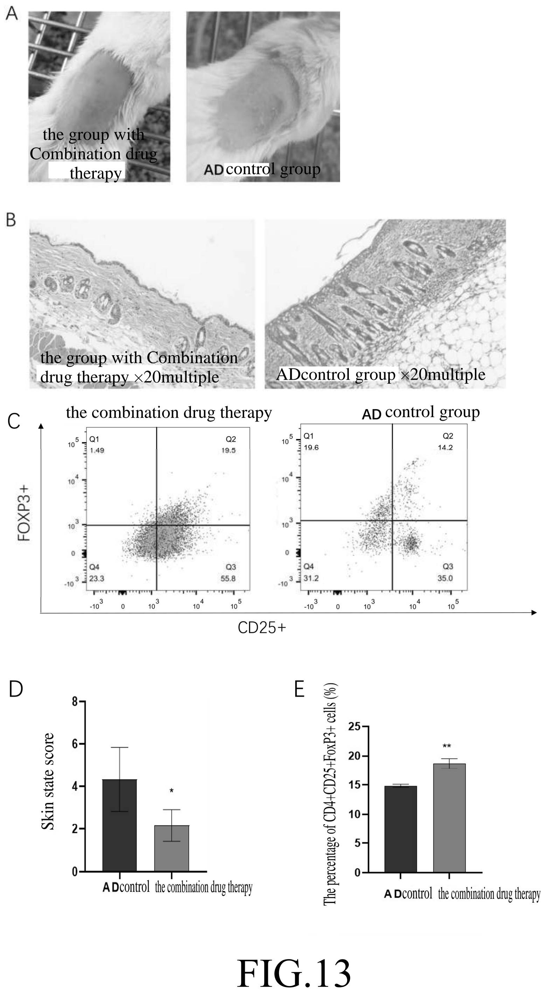

FIG. 13: Detection pictures when contacting OVA patches again after 6 weeks of administration of the combination group and the AD control group. FIG. A is a photograph after 4 days of contacting OVA patches again after the mice in the combination group and the mice in the AD control group were separated from OVA for 6 weeks; FIG. B is respective skin pathological H&E staining images, multiple x20; FIG. C is a flow cytometry detection of CD25+FOXP3+ Treg cells in skin CD4+cells of two groups of mice; FIG. D is a dermatitis score statistical diagram of two groups, and there was a statistical difference between the two groups (P < 0.05); FIG. E is a statistical diagram of Treg cell ratios, and there is a statistical difference between the two groups (P < 0.05), and the study showed that the efficacy of the combination group was maintained for at least 6 weeks. ns represents P > 0.05, * represents P < 0.05, and ** represents P < 0.01.

### Summary of the invention

Unless defined otherwise, all technical and scientific terms have the same meanings as commonly understood by one of ordinary skill in the art. All patents, patent applications, publications, GenBank sequences, web sites, and other disclosure materials are hereby incorporated by reference unless otherwise indicated. In the case of a variety of definitions regarding the terms of the present invention, the present section prevails. When a URL or other identifiers or addresses are described, it should be understood that the identifiers may change and the specific information on the Internet is updated at any time, and the relevant information may be found by searching the Internet. The disclosures thereof are publicly available.

As used herein, the terms "protein", "peptide", "polypeptide" and "amino acid sequence" are used interchangeably and refer to polymers of any length, e.g., two or more amino acid residues. The term also includes amino acid polymers modified naturally or by human intervention; for example, formation of disulfide bonds, glycosylation, esterification, acetylation, phosphorylation, or any other manipulation and modification, such as conjugation to a label or a component having a biological activity. As used herein are conventional one-letter or three-letter representing amino acid residues.

As used herein, the term "amino acid" or "aa" refers to natural and synthetic amino acids as well as amino acid analogs and amino acid mimetics that function in a manner similar to natural amino acids. Natural amino acids are amino acids encoded by genetic codes as well as those modified amino acids later, such as hydroxyproline, γ-carboxyglutamate and O-phosphoserine. Amino acid analogs refer to compounds that have the same basic chemical structure as the native amino acid (i.e., α-carbon associated with hydrogen, carboxyl, amino, and R group). Amino acid mimetics refer to chemical compounds that differ in structure from the general chemical structure of amino acids but function in a manner similar to natural amino acids.

Hyaluronic acid (HA) is a glycosaminoglycan that plays a role in a variety of physiological processes (Laurent TC et al (1992) FASEB J 6:2397-2404). HA is a linear repeat polysaccharide, a linear high molecular weight polysaccharide consisting of repeats of (1-β-4)D-glucuronic acid and (1-β-3)N-acetyl-D-glucosamine as disaccharide unit. HA is present in the extracellular matrix of many cells, particularly in soft connective tissue. In connective tissue, the bound water associated with hyaluronic acid creates space between tissues, thus creating an environment suitable for cell movement and proliferation. Hyaluronic acid plays a role in biological phenomena related to cell viability including rapid development, regeneration, repair, embryogenesis, embryological development, wound healing, angiogenesis, and tumorigenesis (see, e.g., Toole 1991 Cell Biol. Extracell. Matrix, Hay (ed), Plenum Press, New York, 1384-1386; Bertrand et al. 1992 Int. J. Cancer 52:1-6; Knudson et al., 1993 FASEB J. 7:1233-1241). The molecular weight of hyaluronic acid described herein is determined by the addition of each atomic weight of the repeating disaccharide units that make up the hyaluronic acid.

As used herein, large molecular weight HA refers to a hyaluronic acid having a molecular weight of greater than or equal to about 800 KD.

As used herein, small molecular weight HA refers to a hyaluronic acid having a molecular weight of less than or equal to about 300 KD.

As used herein, the molecular weight of HA refers to the number average molecular weight of HA.

The hyaluronic acid used herein can be any suitable form of hyaluronic acid, including hyaluronate, such as, but not limited to, sodium hyaluronate, potassium hyaluronate, and magnesium hyaluronate. In one embodiment, the hyaluronic acid used herein comprises sodium hyaluronate.

The ability of the large molecular weight HA to help drug transdermal absorption and stay on skin is relatively weak, but the large molecular weight HA has immunomodulatory capacity to promote locally activated Treg to express transcription factor FOXP3, enhance Treg immunosuppressive function [FALLACARAA, et al. Hyaluronic Acid in the Third Millennium. Polymers (Basel), 2018, 10 (7): 701; GIRISH K S, KEMPARAJU K. The magic glue hyaluronan and its eraser hyaluronidase: a biological overview. Life Sci, 2007, 80(21): 1921-43; ALTMAN R D, et al. The mechanism of action for hyaluronic acid treatment in the osteoarthritic knee: a systematic review. BMC Musculoskelet Disord, 2015, 16: 321; GUPTA R C, et al. Hyaluronic Acid: Molecular Mechanisms and Therapeutic Trajectory. Front Vet Sci, 2019, 6:192]. With respect to the interaction of HA with TLRs (toll-like receptors), small molecular weight HA has proinflammatory effect and is disadvantageous for allergic dermatitis because it has agonistic activity for TLR-2 and TLR-4, while high molecular weight HA reduces binding capacity to the receptor, forms a dense coating around cells and covers receptor surface, and reduces the pro-inflammatory effect of small molecular weight HA [ALI N, ROSENBLUM M D. Regulatory T cells in skin. Immunology, 2017, 152(3): 372-81; SCHARSCHMIDT T C, et al. A Wave of Regulatory T Cells into Neonatal Skin Mediates Tolerance to Commensal Microbes. Immunity, 2015, 43(5): 1011-21; BILLROTH-MACLURG A C, et al. Regulatory T Cell Numbers in Inflamed Skin Are Controlled by Local Inflammatory Cues That Upregulate CD25 and Facilitate Antigen-Driven Local Proliferation. J Immunol, 2016, 197(6): 2208-18; GRAβHOFF H, et al. Low-Dose IL-2 Therapy in Autoimmune and Rheumatic Diseases. Front Immunol, 2021, 12:648408]

Unless otherwise determined from context, the ratio of large molecular weight HA to small molecular weight HA herein is the mass ratio.

Polyethylene glycol (PEG) is a non-toxic water-soluble neutral polymer, which has good biocompatibility and blood compatibility, and has been used for local human body, gastrointestinal tract and intravenous administration. The PEG modification technology of protein means that one or two end groups at two ends of PEG are activated to have a certain functional group, which functional group is active to at least one functional group in the protein to be bound, and the PEG is bound to the end (N-terminal or C-terminal) or a specific amino acid of the protein by covalent bonding; and the PEG action site is universal.

PEG is a polymer of ethylene glycol and ethylene oxide, having an alias of carbon wax, has a structural formula of:

CH₂(OH)-(CH₂CH₂O)n-CH₂OH

PEG for drug modification may comprise branched and unbranched forms, i.e., linear molecule form (see, e.g. EP0593868) and U/Y-shaped branched molecule form (see, e.g. EP0809996 and CN1243779C).

The molecular formula of the U-type branched PEG derivative (PEG₂-NHS) is as follows: 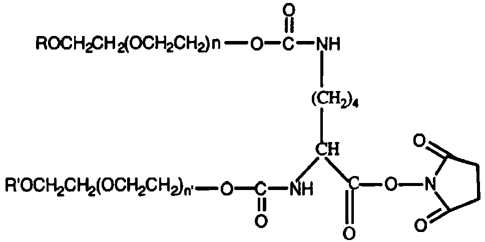 wherein R and R' each are independently low molecular weight alkyl; n and n' are between 600-1500; PEG average molecular weight is between 26 KD and 66 KD.

The Y-type PEG derivative molecule is as follows: 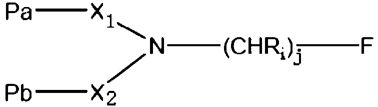 wherein Pₐ and P_{b} are the same or different polyethylene glycol molecules; j is an integer; Rᵢ is H, substituted or unsubstituted alkyl, substituted aryl, aralkyl or heteroalkyl, and the like; X₁ and X₂ each are independently a linking group, such as a group selected from the group consisting of (CH₂)n, (CH₂)ₙOCO, (CH₂)ₙNHCO and (CH₂)ₙCO, in which n is an integer of 1-10, F is an end group, for example, selected from the group consisting of hydroxyl, carboxyl, ester group, acyl chloride, hydrazide, maleimide, pyridine disulfide, and can react with an amino, hydroxyl or thiol group on a therapeutics or matrix to form a covalent bond.

The molecular weight of PEG used herein is determined by the addition of the atomic weight of each atom making up the PEG.

Various methods of determining the molecular weight of a molecule are known in the art. For example, an electrophoretic method can be used to determine the molecular weight of a molecule by aligning with a standard marker. This is within the abilities of those skilled in the art.

As used herein, "glucocorticoid" may be any synthetic or naturally occurring glucocorticoid. Glucocorticoid that may be used in the present invention is, for example, a glucocorticoid useful in the treatment of atopic dermatitis, examples of which include, but not limited to, dexamethasone (Dex), budesonide (Bud), beclomethasone dipropionate (BDP), ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, and tipredane.

In the context of the present specification, unless otherwise specified, any reference to "glucocorticoid" includes all active salts, solvates or derivatives that can be derived from the glucocorticoid, for example, reference to "dexamethasone" encompasses dexamethasone and its active salts, solvates or derivatives having desired activity. Examples of possible salts or derivatives of glucocorticoid include sodium salt, sulfobenzoate, phosphate, isonicotinate, acetate, propionate, dihydrogen phosphate, palmitate, pivalate, fumarate, and pharmaceutically acceptable esters (e.g., C1-C6 alkyl esters). Glucocorticoid and its active salts or derivatives may also be in their solvated forms, such as hydrated forms.

As used herein, "IL-2" refers to IL-2 of any source, including mammalian sources such as humans, mice, rats, primates, and pigs, and may be natural or obtained by recombinant or synthetic techniques, including recombinant IL-2 polypeptides produced by microbial hosts. IL-2 may be or comprises a natural polypeptide sequence, or may be an active variant of a natural IL-2 polypeptide. Preferably, IL-2 polypeptide or active variant is derived from a human source and comprises recombinant human source IL-2, particularly recombinant human source IL-2 produced by a microbial host.

IL2 that can be used in the present invention is any clinically useful IL2 for the treatment of type I allergic reactions, particularly atopic dermatitis. Those skilled in the art can obtain or determine which IL2 or derivatives or variants thereof can be used in the present invention.

As used herein, PEG modified IL-2 refers to IL-2 modified by linking a PEG such as unbranched PEG or branched PEG to any suitable site on IL-2.

As used herein, "treating" a subject having atopic dermatitis refers to partial or complete elimination of a lesion or symptom of the subject, or remaining stable after treatment without progression. Treatment includes prevention, curing and/or healing. Prevention refers to preventing a potential disease from occurring and/or worsening or progression, preventing the occurring includes alleviating or eliminating one or more risk factors that cause disease to occur; because it is generally not possible to determine whether a disease has never occurred, thus prevention also includes lowering a risk of occurrence of or suffering from a disease.

As used herein, "therapeutically effective amount" or "therapeutically effective dose" refers to a dose that is at least sufficient to produce a therapeutic effect in a subject. For the active ingredients herein, specific therapeutically effective amounts may be initially estimated using a variety of techniques known in the art. Dosage ranges suitable for human subjects may be determined, for example, using data from cell culture experiments and other animal studies. Dosage levels and protocols may be determined based on known dosages and protocols, if desired, based on known properties and/or may be determined empirically based on a variety of factors. Such factors include, for example, the weight of the subject, overall health, age, activity of the particular compound used, sex, diet, time of administration, drug combination, disease severity and course, and the patient's disease susceptibility and physician judgment. After the patient condition is improved, a maintenance dose of the compound or composition may be administered, and if desired, the dosage, dosage form, and frequency of administration, or combinations thereof, may vary. The precise dosage and regimen should depend on the physician's discretion and the particular patient's condition.

The therapeutically effective amount depends largely on the nature of the drug, the patient's condition, and the nature and severity of the condition to be treated. The therapeutically effective amount may range from as low as 1 ng/kg, for example, when a topical condition such as atopic dermatitis is treated, an effective active is used, to as much as 10 mg/kg, more particularly between 20 ng/kg and 1 mg/kg.

Herein, sensitized BALB/c mice were intraperitoneally injected with chicken ovalbumin (OVA) three times, and typical Type I hypersensitivity responses were induced by OVA challenge at different sites, exhibiting skin damage and high airway response, high serum IgE, skin pathological inflammatory cell infiltration, Th2 cell polarization and high IL-4 and IL-3 expression. OVA sensitized BALB/c mice with induced type I allergy are the most common experimental animal models. On the basis of the OVA-induced atopic dermatitis mice model, firstly, PEG-IL-2 in combination with budesonide was topically administered, and compared with the therapeutic effect of the current clinical first-line treatment protocol (i.e., local administration of a glucocorticoid only), both treatment regimens were found to relieve the symptoms of dermatitis in mice, which upregulated the proportion of Treg cells in the skin to a certain extent, but the therapeutic effect of the combination was not improved compared with the efficacy of only budesonide, and there was no statistical difference in Treg cell proportion in the skin. As there is budesonide in the combination, alleviation of dermatitis should result from the action of glucocorticoid.

The core of the present study was to correct skin immune imbalance by upregulating the proportion of Tregs in the skin while enhancing their immunosuppressive ability with medication so as to treat and prevent atopic dermatitis.

In normal skin structure, a substance with a molecular weight of more than 4 kD is difficult to penetrate stratum corneum into epidermis and dermis layers, especially when the substance is in hydrophilic state. This study showed that the combination administered after allergen sensitization but dermatitis (skin lesion) had not occurred, cannot prevent the OVA allergen from inducing dermatitis, indicating that HA cannot assist the drugs in permeating into the skin of normal structure. As shown herein, in the mouse model of atopic dermatitis e.g. in which stratum corneum was damaged, in the case that skin is in inflammatory state, the therapeutic effect of PEG-IL-2 combined budesonide together with a small molecular weight hyaluronic acid was significantly improved, and the same time Treg cells were up-regulated, indicating that a small molecular weight hyaluronic acid can help a large molecular weight drug having hydrophilic polarity to penetrate into the deep of epidermis or dermis layers or remain in the deep of the epidermis or dermis layers (deep of epidermis or dermis layers, is the responsive layer of atopic dermatitis immunity). The smaller the molecular weight of the combined PEG-IL-2 (PEG of 10 kDa, 20 kDa and 40 kDa), the more the proporation of up-regulated Treg cells in the skin, the better the therapeutic effect of dermatitis, the efficacy of 10kD-IL-2 (molecular weight 25 kDa) is better than 20kD-IL-2 (molecular weight of 35 kDa) or 40kD-IL-2 (molecular weight 55 kDa), but when replacing the PEG-IL-2 with normal interleukin 2 (molecular weight 15 kDa) in the combination regimen, the molecular weight was reduced and the hydrophilic polarity of PEG was removed (the polarity reduction favors drug permeation), and the results showed that the increase of induced Treg cells and the improvement of skin inflammation were not as good as the PEGylated interleukin 2, and had the same therapeutic efficacy as treatment with budesonide alone, so it appears that the normal interleukin 2 cannot be permeate into the damaged skin to the deep layer of the epidermis by the small molecular weight hyaluronic acid, but Madeleine Witting et al. [WARD-HARTSTONGE K A, KEMP R A. Regulatory T-cell heterogeneity and the cancer immune response. Clin Transl Immunology, 2017, 6(9): e154; DONG L, et al. Programmed death 1/programmed cell death-ligand 1 pathway participates in gastric surgery-induced imbalance of T-helper 17/regulatory T cells in mice. J Trauma Acute Care Surg, 2018, 85(3): 549-59] showed that all calf serum albumin BSA of molecular weight over 60 kD can reach the epidermis through the damaged skin.

In the damaged skin state, we also used 10kD PEG-IL-2 (interleukin 2 modified with such PEG molecular weight had a bias to induce Treg proliferation) for external application on the damaged skin, and also showed therapeutic effects, but the efficacy was worse than that of the small molecular weight HA combined with 10kD PEG-IL-2, indicating that PEG-IL-2 could permeate into the skin in damaged skin state, therefore, interleukin 2 not modified with PEG could also enter the skin through the stratum corneum of damaged skin, while, under the action of HA, cannot reach or stay in the deep layer of epidermis or dermis layer where immune response occurs, while interleukin 2 was limited to the superficial layer of the epidermis under the action of the small molecular weight HA, while the PEG-modified interleukin 2 could penetrate into the deep layer of epidermis or dermis layer of the skin through the small molecular weight hyaluronic acid to achieve the therapeutic effect. It is believed that the drug stayed in the active deep layer of epidermis or dermis layer due to the interaction between the small molecular weight HA and PEG accounts for the better effects, while, in the absence of PEG, the small molecular weight HA makes the drug to stay in the superficial layer of the epidermis, but not in the site where immune response occurs.

Without wishing to be bound by any theory, the experiments provided herein illustrate that a PEG-modified IL-2 combined a glucocorticoid, with a molecular weight varied within a certain range, does not have a major effect on therapeutic effect on skin. It is possible that the hyaluronic acid and the PEG molecule are bound in some form, which can help PEG-IL-2 enter and/or stably remain in the deep layer of epidermis or dermis layer to exert therapeutic effects, while the normal IL-2 without binding to a PEG molecule cannot enter the deep layer of epidermis or dermis layer with the help of HA, or can only transiently stay in the epidermis, and will lose before an effective action is exerted. The specific binding manner between PEG and HA molecules is not clear, and there is no related report in respect of the interaction therebetween.

There was no statistical difference between the 10kD branched PEG-IL-2 group and 10kD unbranched PEG-IL-2 group, in dermatitis manifestation, inflammatory cell infiltration or Treg ratio in skin, indicating that the coupling of PEG of different structures with IL-2 had no significant effect on overall efficacy. Studies herein showed that changes in molecular weight and structure of PEG have little relationship to the entry and retention of drugs in the deep layer of epidermis or dermal layers.

Why Tregs cells induced by and therapeutic effects of 10kD-IL-2 combined glucocorticoid and small molecular weight HA are better than 20kD or 40kD PEG-IL-2 groups? Without wishing to be bound by any theory, it is likely that besides PEG-modified interleukin 2 had improved skin penetration due to interaction with hyaluronic acid, modification with PEG of specific molecular weights may also alter the performance of interleukin 2. When BALB/c mice in the physiological state were injected with interleukin 2 modified with unbranched PEG of different molecular weights, 10kD, 20kD and 40kD, and normal IL-2, it was unexpectedly found that only interleukin 2 modified with PEG having a molecular weight of 10kD predominantly induced Treg cell proliferation and the PD-1 molecule was highly expressed on induced Treg cells, and Treg cells highly expressing PD-1 enhanced inhibitory effect of Treg on effector T cells (Teff) [PARK H J, PARK J S, JEONG Y H, et al. PD-1 upregulated on regulatory T cells during chronic virus infection enhances the suppression of CD8+ T cell immune response via the interaction with PD-L1 expressed on CD8+ T cells [J]. J Immunol, 2015, 194(12): 5801-11].

Herein, the symptoms of mouse dermatitis can be improved when using 10kD PEG IL-2 with or without HA (not combined glucocorticoid), and the proportion of Treg cells in the skin of mice after treatment was increased to some extent compared with that in dermatitis model mice, and the difference is statistically significant, while 40kD PEG-IL-2 alone had no therapeutic effect (FIG. 3). Therefore, the inventors believe that 10kD PEG-IL-2 itself is a novel interleukin 2 capable of prodominantly inducing Treg cells proliferation, while the interleukin 2 modified with 20kD or 40kD PEG needs to combine a glucocorticoid to prodominantly induce Treg cell proliferation. Obviously, modification with PEG of suitable molecular weights can lead to changes in biological activity of interleukin-2 for prodominantly inducing Treg.

In addition, when 10kD PEG-IL-2 was applied in the mouse asthma model, the airway hyperreactivity of the mouse was significantly reduced, while, on this basis, addition of HA cannot further improve airway hyperresponsiveness, therefore, it is believed that 10kD PEG-IL-2 can alleviate the type I allergy, alleviate symptoms of various type I allergy-related diseases, and again prove that 10kD PEG-IL-2 is a novel IL-2 having an immunomodulatory function.

Subsequently, in the case the 10kD PEG-IL-2 in combination with budesonide was selected, the size of HA molecular weight and the ratio of HA were varied, and the proportion of Tregs in skin was found to be the highest in the groups of a large molecular weight HA/a small molecular weight HA at a ratio of 1:2 and the group of a small molecular weight HA merely. It is believed that both the large molecular weight HA and the small molecular weight HA can enhance therapeutic effect of 10kD PEG-IL-2 in combination with budesonide, but the assistive effect of the small molecular weight HA is greater because the small molecular weight HA can more easily bring the combined drugs into and stay on the skin active layer, so the assistive effect of the small molecular wegith HA becomes stronger as it increases, and the therapeutic effect is better, so when the amount of the small molecular weight HA is sufficient to bring most drugs into skin, the therapeutic effect is no longer improved.

Therefore, the best effect can be achieved when the large molecular weight HA and the small molecular weight HA are at an appropriate ratio. Finally, a combination of 10kDa PEG-IL-2+budesonide+1/3 large molecular weight HA+2/3 small molecular weight HA is selected as the most preferred solution of the study. By further comparison with the clinical first-line treatment regimens, it is proved that the optimal solution determined by the study can more quickly and effectively alleviate dermatitis symptoms, downregulate inflammatory factors and serum IgE concentration, upregulate the proportion of Treg cells in skin to a greater extent, and still prevent relapse of dermatitis after 6 weeks of administration, and the proportion of Treg cells in skin is also maintained at a higher level, and this long-term efficacy may also be related to long-term stable stay of the induced Treg cells by HA in the skin, so the HA may interact with Treg cells, and the Treg cells are stayed in dermis layer, and the Treg cells living in dermis layer for long term may automatically regulate the immune response in the skin to achieve long-term therapeutic effects, and can prevent the onset of dermatitis after contacting allergen again in allergen-sensitized skin, and prevent allergies such as seasonal pollen.

In summary, the 3-day short course of topically applying the combination scheme of 10kDa PEG-IL-2+budesonide (or dexamethasone)+1/3 large molecular weight HA+2/3 small molecular weight HA can quickly and effectively alleviate and prevent atopic dermatitis in mice, reduce the infiltration of local inflammatory cells, up-regulate the proportion of Treg cells in skin, and the effect lasts for at least 6 weeks. In this scheme, it is unexpectedly found that PEG molecules interact with HA of small molecular weight, which solves the problem that the hydrophilic polar and large molecular weight protein drugs are difficult to penetrate or remain in the deep layer of epidermis or dermis layers in skin, and unexpectedly found that the modification of interleukin 2 with PEG of 10kD molecular weight alters the biological characteristics of interleukin 2, and between Treg cells (inhibiting allergic immunity) and effector cells (causing allergic reactions), Treg cells can be expanded predominantly to alleviate type I allergy reactions, so 10kD PEG-IL-2 is a novel interleukin 2.

Without wishing to be bound by any theory, the possible mechanism is that 10kD PEG-IL-2 is capable of predominantly proliferating Tregs and promoting high expression of PD-1 thereof to enhance the immunosuppressive function, while the bias protection on Treg cells withstand glucocorticoid-induced apoptosis, while the small molecular weight HA can assist in drug absorption, and binding with PEG molecules in some form helps the drug to stay stably in the deep layer of epidermis and dermal layers, and may also stabilize Treg cells to stay in the deep layer of epidermis and dermal layers of skin to achieve long-term stable local immunity to produce persistent therapeutic effects, while the function of large molecular weight HA to enhance Treg immunomodulation at the same time inhibits occurrence of allergic immunity.

Thus, in a first aspect, there is provided a pharmaceutical composition comprising a polyethylene glycol-modified interleukin 2 (PEG-IL2), a glucocorticoid and a small molecular weight hyaluronic acid (HA), and optionally a pharmaceutically acceptable carrier.

The PEG used to modify IL2 can be any PEG of suitable type or size, such as linear or non-linear PEG, and/or the size is any suitable size.

In one embodiment, polyethylene glycol (PEG) for modifying IL-2 is a PEG having a molecular weight of less than or equal to about 100, 90, 80, 70, 60, 55, 50, 45, 40, 35, 30, 25, 20, 15, 10 or 5 KD. In one embodiment, the PEG is a PEG having a molecular weight of about 1-100, 1-90, 1-80, 1-70, 1-60, 1-50, 1-40, 1-10, 1-20, 2-100, 2-90, 2-80, 2-70, 2-60, 2-50, 2-40, 2-30, 2-20, 3-100, 3-90, 3-80, 3-70, 3-60, 3-50, 3-40, 3-30, 3-20, 4-100, 4-90, 4-80, 4-70, 4-60, 4-50, 4-40, 4-30, 4-20, 5-100, 5-90, 5-80, 5-70, 5-60, 5-50, 5-40, 5-30, 5-20, 10-100, 10-90, 10-80, 10-70, 10-60, 10-50, 10-40, 10-30 or 10-20 KD.

In one embodiment, the PEG is a PEG having a molecular weight of about 60 KD, 55 KD, 50 KD, 45 KD, 40 KD, 35 KD, 30 KD, 25 KD, 20 KD, 15 KD, 10 KD, or 5 KD.

In one embodiment, the PEG comprises branched or unbranched PEG. For example, the branched PEG may comprise branched PEG of about 10 KD and 20 KD; the unbranched PEG comprises unbranched PEG of about 10 KD, 20 KD, and 40 KD.

The PEG modification described herein can be at any site suitable for PEG modification in IL-2, for example, lysine, serine, threonine residues or N-terminal α-amino group at IL-2. In one embodiment, the PEG modification is made at the N-terminal amino acid residue of IL-2, for example, the N-terminal lysine, serine, or threonine residue of IL-2. In one embodiment, the PEG modification is at the N-terminal α-amino group of IL-2. The PEG modification may be, for example, a single-site modification or multi-site modification, preferably a single-site modification.

In one embodiment, the IL-2 is an IL-2 from human or non-human mammals, for example, bovine, rat, mouse, dog, monkey, goat, sheep, cow, deer, horse and cat.

In one embodiment, an IL-2 of human source or an active variant thereof is used herein, more preferably produced by recombination. The nucleotide and amino acid sequences of the IL-2 of human source are disclosed, for example, in Genbank ref 3558 or P60568, respectively. Unless otherwise specified, the IL-2 used is in substantially pure form, for example, with a purity of 95% or more, more preferably 96%, 97%, 98% or 99% pure. IL-2 can be used in the form of monomeric protein or multimeric protein.

In one embodiment, IL2 as used herein comprises the amino acid sequence of SEQ ID NO: 1.

The small molecular weight HA refers to a HA having a molecular weight of less than or equal to about 300 KD, such as less than or equal to about 250, 200, 150, 100, 90, 80, 70, 60, 50, 40, 30, 20, 10, 9, 8, 7, 6, 5, 4, 3 or 2 KD. In one embodiment, the small molecular weight HA refers to a HA having a molecular weight of about 2-20, 2-15, 2-10, 2-5, 3-20, 3-15, 3-10, or 3-5 KD, e.g., about 5 KD, 4 KD, 3 KD, or 2 KD.

In one embodiment, the pharmaceutical composition further comprises a large molecular weight HA, wherein the large molecular weight HA and the small molecular weight HA can be present in any ratio, provided that the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less. Preferably, the pharmaceutical composition comprises a large molecular weight HA and a small molecular weight HA at a mass ratio of about 2:1 or about 1:2.

The large molecular weight HA refers to a HA having a molecular weight of greater than or equal to about 800 KD, such as greater than or equal to about 900, 1000, 1100, 1200, 1300, 1400, 1500, 1600, 1700, 1800, 1900, 2000 KD or greater. In one embodiment, the large molecular weight HA refers to a HA having a molecular weight of about 800-2000, 800-1900, 800-1800, 800-1700, 800-1600, 800-1500, 900-2000, 900-1900, 900-1800, 900-1700, 900-1600, 900-1500, 1,000-2000, 1,000-1900, 1,000-1800, 1,000-1700, 1000-1600, or 1000-1500 KD, in particular, a HA of about 800-1500 KD.

In one embodiment, the glucocorticoid is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof. In one embodiment, the glucocorticoid comprises dexamethasone, budesonide, and derivatives thereof.

In one embodiment, the pharmaceutical composition is used to treat atopic dermatitis. Preferably, atopic dermatitis is atopic dermatitis with skin lesions. As used herein, atopic dermatitis with skin lesions refers to atopic dermatitis where the structure of the skin stratum corneum is damaged, such as damaged keratinized membrane and lipid membrane and stratum granulosum, the final defense-line of skin barrier, manifested as chronic and relapsed severe itching, erythema, edema, blisters, serous exudation, and the like.

In one embodiment, provided herein is a pharmaceutical composition comprising:
- 10KD PEG modified IL2,
- dexamethasone or budesonide, and
- a HA, comprising: (i) a small molecular weight HA, or (ii) a small molecular weight HA and a large molecular weight HA, wherein the mass ratio of the large molecular weight HA to the small molecular weight HA is about 1:2.

As used herein, "10KD PEG modified IL2" is an IL-2 modified with a PEG having a molecular weight of about 10KD, wherein the PEG can be a PEG of any suitable type, such as linear or nonlinear PEG. The "about 10 KD" means the sum of the atomic weights of the atoms constituting the PEG is 10 KD ± 10%, 10 KD ± 5%, 10 KD ± 4%, 10 KD ± 3%, 10 KD ± 2%, 10 KD ± 1%, 10 KD ± 0.5%, or even 10 KD ± 0.1%. In a preferred embodiment, the 10KD PEG modified IL2 is an IL-2 (for example, SEQ ID NO: 1) modified with a PEG having a molecular weight of about 10 KD at the N-terminus of the IL-2.

The pharmaceutical composition may further comprise a pharmaceutically acceptable carrier. The term "pharmaceutically acceptable carrier" includes any and all solvents, dispersion media, coatings, antibacterial and antifungal agents, isotonic agents and absorption delaying agents, and the like. The use of such media and agents is well known in the art. As used herein, active ingredients such as glucocorticoid and polyethylene glycol-modified interleukin 2 may be used in admixture with one or more pharmaceutically acceptable additives, diluents, or carriers. Examples of pharmaceutically acceptable carrier include, for example, but not limited to, lactose, sucrose, dextran, mannitol or glucose starch, talc, magnesium stearate, magnesium oxide, crystalline cellulose, methylcellulose, carboxymethylcellulose, gelatin, glycerol, sodium alginate, saline and water, and may also contain additives such as fillers, binders, moisturizers, glidants, stabilizers, preservatives, emulsifiers and additional solvents or solubilizers or substances that achieve storage effects.

The pharmaceutical compositions may be formulated in any suitable form for topical administration (such as locally or topically), such as powders, sprays, foams, solutions, ointments, emulsions, and the like. The pharmaceutical composition may be formulated as a solid, liquid, gel, or other forms.

The pharmaceutical compositions may be provided in the form of bulk and unit doses and in the form of an openable or puncturable implant, capsule, blister pack or cartridge known in the art. Also provided is a kit containing a delivery device, a pharmaceutical composition contained herein in a separate container, and optionally other suitable additives, such as other therapeutic compounds, excipients, surfactants (used as therapeutic drugs and formulation ingredients), antioxidants, flavoring and coloring agents, fillers, volatile oils, buffers, dispersing agents, surface active substances, antioxidants, flavoring agents, bulking agents, propellants and preservatives, and instructions for using the kit components.

In a second aspect, there is provided a method for treating atopic dermatitis in a subject, comprising administering to the subject a therapeutically effective amount of PEG-IL2, a glucocorticoid and a small molecular weight HA.

The PEG-IL2 administered to the subject can be an IL-2 modified with a PEG of any suitable type or size, such as linear or non-linear PEG and/or PEG of any suitable size.

In one embodiment, the PEG-IL2 administered to the subject is modified with a PEG having a molecular weight of less than or equal to about 100, 90, 80, 70, 60, 55, 50, 45, 40, 35, 30, 25, 20, 15, 10 or 5 KD. In one embodiment, the PEG is a PEG having a molecular weight of about 1-100, 1-90, 1-80, 1-70, 1-60, 1-50, 1-40, 1-10, 1-20, 2-100, 2-90, 2-80, 2-70, 2-60, 2-50, 2-40, 2-30, 2-20, 3-100, 3-90, 3-80, 3-70, 3-60, 3-50, 3-40, 3-30, 3-20, 4-100, 4-90, 4-80, 4-70, 4-60, 4-50, 4-40, 4-30, 4-20, 5-100, 5-90, 5-80, 5-70, 5-60, 5-50, 5-40, 5-30, 5-20, 10-100, 10-90, 10-80, 10-70, 10-60, 10-50, 10-40, 10-30 or 10-20 KD.

In one embodiment, the PEG is a PEG having a molecular weight of about 60 KD, 55 KD, 50 KD, 45 KD, 40 KD, 35 KD, 30 KD, 25 KD, 20 KD, 15 KD, 10 KD, or 5 KD.

In one embodiment, the PEG comprises branched or unbranched PEG. For example, the branched PEG may comprise a branched PEG of about 10 KD and 20 KD; the unbranched PEG comprises unbranched PEG of about 10 KD, 20 KD, and 40 KD.

In one embodiment, the PEG modification is at the N-terminal amino acid residue of IL-2, for example, the lysine, serine, and threonine residues at the N-terminus of IL-2. In one embodiment, the PEG modification is at the N-terminal α-amino group of IL-2. The PEG modification may be, for example, single-site modification or multi-site modification, preferably single-site modification.

In one embodiment, the IL-2 is an IL-2 of human or non-human mammals such as, for example, bovine, rat, mouse, dog, monkey, goat, sheep, cow, deer, horse, and cat.

In one embodiment, IL2 as used herein comprises the amino acid sequence of SEQ ID NO: 1.

The small molecular weight HA refers to a HA having a molecular weight of less than or equal to about 300 KD, such as about 250, 200, 150, 100, 90, 80, 70, 60, 50, 40, 30, 20, 10, 9, 8, 7, 6, 5, 4, 3 or 2 KD. In one embodiment, the small molecular weight HA refers to a HA having a molecular weight of about 2-20, 2-15, 2-10, 2-5, 3-20, 3-15, 3-10, or 3-5 KD, e.g., about 5 KD, 4 KD, 3 KD, or 2 KD. In one embodiment, the small molecular weight HA refers to a HA having a molecular weight of about 4 KD.

In one embodiment, the method comprises administering a small molecular weight HA having a molecular weight of less than or equal to about 5 KD, e.g., about 4 KD, 3 KD, or 2 KD. In one embodiment, the small molecular weight HA refers to a HA having a molecular weight of about 4 KD.

In one embodiment, the method further comprises administering a large molecular weight HA to the subject, wherein the large molecular weight HA and the small molecular weight HA can be administered at any ratio, provided that the mass ratio of the large molecular weight HA to the small molecular weight HA as administered is about 2:1, or that the mass of the administered large molecular weight HA is less than 50%, preferably less than 40%, 35%, 30%, 25%, 20%, 15%, 10% or less, based on the total mass of the administered large molecular weight HA and small molecular weight HA. Preferably, the HA administered to the subject comprises a large molecular weight HA and a small molecular weight HA at a mass ratio of about 1:2.

The large molecular weight HA refers to a HA having molecular weight of greater than or equal to about 800 KD, such as greater than or equal to about 900, 1000, 1100, 1200, 1300, 1400, 1500, 1600, 1700, 1800, 1900, 2000 KD or greater. In one embodiment, the large molecular weight HA refers to a HA having a molecular weight of about 800-2000, 800-1900, 800-1800, 800-1700, 800-1600, 800-1500, 900-2000, 900-1900, 900-1800, 900-1700, 900-1600, 900-1500, 1,000-2000, 1,000-1900, 1,000-1800, 1,000-1700, 1000-1600, or 1000-1500 KD, in particular, a HA of about 800-1500 KD.

In one embodiment, the glucocorticoid administered to the subject is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof. In one embodiment, the glucocorticoid comprises dexamethasone, budesonide, or a derivative thereof.

In one embodiment, the PEG-IL2, glucocorticoid and HA may be applied to the diseased site simultaneously or sequentially in any order.

In one embodiment, the method comprises administering to the subject a therapeutically effective amount of the pharmaceutical composition of the first aspect.

As used herein, the subject is a human subject or non-human subject having atopic dermatitis, and non-limiting examples include humans, other mammals such as cows, rats, mice, dogs, monkeys, goats, sheep, cows, deer, horses, cats, and other non-mammals. In some embodiments, the subject is a human.

In one embodiment, atopic dermatitis of the subject is atopic dermatitis with skin lesions. In one embodiment, the method comprises administering to the subject:
- 10KD PEG modified IL2,
- dexamethasone or budesonide, and
- HA, comprising: (i) a small molecular weight HA, or (ii) a small molecular weight HA and a large molecular weight HA, wherein the mass ratio of the large molecular weight HA to the small molecular weight HA is about 1:2.

In one embodiment, the method comprises administering to the subject a pharmaceutical composition comprising:
- 10KD PEG modified IL2,
- dexamethasone or budesonide, and
- HA, comprising: (i) a small molecular weight HA, or (ii) a small molecular weight HA and a large molecular weight HA, wherein the mass ratio of the large molecular weight HA to the small molecular weight HA is about 1:2.

As used herein, administration refers to topically administering (e.g., locally or topically) the active or pharmaceutical composition to a diseased site of atopic dermatitis. Administration can be performed using any method known in the art as well as adjunctive means, such as by direct application, spray, drip, patch, etc.

In a third aspect, provided is use of a PEG-IL2, a glucocorticoid and a small molecular weight HA in the preparation of a medicament for the treatment of atopic dermatitis (in particular, the medicament is for topical or surface external application) or kits; and a PEG-IL2, a glucocorticoid and a small molecular weight HA for treating atopic dermatitis.

The small molecular weight HA refers to a HA having a molecular weight of less than or equal to about 300 KD, such as about 250, 200, 150, 100, 90, 80, 70, 60, 50, 40, 30, 20, 10, 9, 8, 7, 6, 5, 4, 3 or 2 KD. In one embodiment, the small molecular weight HA refers to a HA having a molecular weight of about 2-20, 2-15, 2-10, 2-5, 3-20, 3-15, 3-10, or 3-5 KD, e.g., about 5 KD, 4 KD, 3 KD, or 2 KD. In one embodiment, the small molecular weight HA refers to a HA having a molecular weight of about 4 KD.

In one embodiment, the PEG-IL2 is a PEG having a molecular weight of less than or equal to about 100, 90, 80, 70, 60, 55, 50, 45, 40, 35, 30, 25, 20, 15, 10 or 5 KD. In one embodiment, the PEG is a PEG having a molecular weight of about 1-100, 1-90, 1-80, 1-70, 1-60, 1-50, 1-40, 1-10, 1-20, 2-100, 2-90, 2-80, 2-70, 2-60, 2-50, 2-40, 2-30, 2-20, 3-100, 3-90, 3-80, 3-70, 3-60, 3-50, 3-40, 3-30, 3-20, 4-100, 4-90, 4-80, 4-70, 4-60, 4-50, 4-40, 4-30, 4-20, 5-100, 5-90, 5-80, 5-70, 5-60, 5-50, 5-40, 5-30, 5-20, 10-100, 10-90, 10-80, 10-70, 10-60, 10-50, 10-40, 10-30 or 10-20 KD.

In one embodiment, the PEG is a PEG having a molecular weight of about 60 KD, 55 KD, 50 KD, 45 KD, 40 KD, 35 KD, 30 KD, 25 KD, 20 KD, 15 KD, 10 KD, or 5 KD.

In one embodiment, the PEG comprises branched or unbranched PEG. For example, the branched PEG may comprise a branched PEG of about 10 KD and 20 KD; the unbranched PEG comprises unbranched PEG of about 10 KD, 20 KD, and 40 KD.

In one embodiment, the PEG modification is at the N-terminal amino acid residue of IL-2, for example, the lysine, serine, or threonine residue at the N-terminus of IL-2. In one embodiment, the PEG modification is at the N-terminal α-amino group of IL-2. The PEG modification may be, for example, a single-site modification or multi-site modification, preferably a single-site modification.

In one embodiment, the IL-2 is an IL-2 of human or non-human mammal such as, for example, bovine, rat, mouse, dog, monkey, goat, sheep, cow, deer, horse, cat, and the like.

In one embodiment, IL2 as used herein comprises the amino acid sequence of SEQ ID NO: 1.

In one embodiment, further provided is use of a PEG-IL2, a glucocorticoid, a large molecular weight HA and a small molecular weight HA in the preparation of a medicament or kit for treating atopic dermatitis; and a PEG-IL2, a glucocorticoid, a large molecular weight HA and a small molecular weight HA for use in the treatment of atopic dermatitis, wherein the large molecular weight HA and the small molecular weight HA can be at any ratio, provided that the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less. Preferably, the mass ratio of the large molecular weight HA to the small molecular weight HA is about 1:2.

The large molecular weight HA refers to a HA having a molecular weight of greater than or equal to about 800 KD, such as greater than or equal to about 900, 1000, 1100, 1200, 1300, 1400, 1500, 1600, 1700, 1800, 1900, 2000 KD or greater. In one embodiment, the large molecular weight HA refers to a HA having a molecular weight of about 800-2000, 800-1900, 800-1800, 800-1700, 800-1600, 800-1500, 900-2000, 900-1900, 900-1800, 900-1700, 900-1600, 900-1500, 1,000-2000, 1,000-1900, 1,000-1800, 1,000-1700, 1000-1600, or 1000-1500 KD, in particular, a HA of about 800-1500 KD.

In one embodiment, the glucocorticoid is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof. In one embodiment, the glucocorticoid comprises dexamethasone, budesonide, or a derivative thereof.

In one embodiment, the atopic dermatitis is atopic dermatitis with skin lesions.

In a fourth aspect, provided is a kit comprising a PEG-IL2, a glucocorticoid and a small molecular weight HA.

The kit may further comprise other reagents for formulating the contained PEG- IL2, glucocorticoid and HA as appropriate agents (e.g., solvents, buffers, etc.) and optionally an instruction.

In one embodiment, for PEG-IL2 is an IL2 modified with a PEG having a molecular weight of less than or equal to about 100, 90, 80, 70, 60, 55, 50, 45, 40, 35, 30, 25, 20, 15, 10 or 5 KD. In one embodiment, the PEG is a PEG having a molecular weight of about 1-100, 1-90, 1-80, 1-70, 1-60, 1-50, 1-40, 1-10, 1-20, 2-100, 2-90, 2-80, 2-70, 2-60, 2-50, 2-40, 2-30, 2-20, 3-100, 3-90, 3-80, 3-70, 3-60, 3-50, 3-40, 3-30, 3-20, 4-100, 4-90, 4-80, 4-70, 4-60, 4-50, 4-40, 4-30, 4-20, 5-100, 5-90, 5-80, 5-70, 5-60, 5-50, 5-40, 5-30, 5-20, 10-100, 10-90, 10-80, 10-70, 10-60, 10-50, 10-40, 10-30 or 10-20 KD.

In one embodiment, the PEG is a PEG having a molecular weight of about 60 KD, 55 KD, 50 KD, 45 KD, 40 KD, 35 KD, 30 KD, 25 KD, 20 KD, 15 KD, 10 KD or 5 KD.

In one embodiment, the PEG comprises branched or unbranched PEG. For example, the branched PEG may comprise branched PEG of about 10 KD and 20 KD; and the unbranched PEG comprises unbranched PEG of about 10 KD, 20 KD and 40 KD.

The PEG modification described herein can be at any site in IL-2 suitable for PEG modification, for example, at lysine, serine, threonine residue or N-terminal α-amino group at IL-2. In one embodiment, the PEG modification is at the N-terminal amino acid residue of IL-2, for example, the lysine, serine or threonine residue at the N-terminus of IL-2. In one embodiment, the PEG modification is at the N-terminal α-amino group of IL-2. The PEG modification may be, for example, a single-site modification or multi-site modification, preferably a single-site modification.

In one embodiment, the IL-2 is an IL-2 from human or non-human mammals such as, for example, bovine, rat, mouse, dog, monkey, goat, sheep, cow, deer, horse, cat, and the like.

In one embodiment, an IL-2 of human source or an active variant thereof is used herein, more preferably produced by recombination. The nucleotide and amino acid sequences of human source IL-2 are disclosed, for example, in Genbank ref 3558 or P60568, respectively. Unless otherwise specified, the IL-2 used is in substantially pure form, for example, with a purity of 95% or more, more preferably 96%, 97%, 98% or 99% pure. IL-2 can be used in the form of monomeric protein or multimeric protein.

In one embodiment, IL2 as used herein comprises the amino acid sequence of SEQ ID NO: 1.

The small molecular weight HA refers to a HA having a molecular weight of less than or equal to about 300 KD, such as about 250, 200, 150, 100, 90, 80, 70, 60, 50, 40, 30, 20, 10, 9, 8, 7, 6, 5, 4, 3 or 2 KD. In one embodiment, the small molecular weight HA refers to a HA having a molecular weight of about 2-20, 2-15, 2-10, 2-5, 3-20, 3-15, 3-10 or 3-5 KD, e.g., about 5 KD, 4 KD, 3 KD or 2 KD. In one embodiment, the small molecular weight HA refers to a HA having a molecular weight of about 4 KD.

In one embodiment, the kit further comprises a large molecular weight HA, wherein the large molecular weight HA and the small molecular weight HA are present at any ratio, provided that the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less. Preferably, the kit comprises a large molecular weight HA and a small molecular weight HA at a mass ratio of about 1:2.

The large molecular weight HA refers to a HA having a molecular weight of greater than or equal to about 800 KD, such as greater than or equal to about 900, 1000, 1100, 1200, 1300, 1400, 1500, 1600, 1700, 1800, 1900, 2000 KD or greater. In one embodiment, the large molecular weight HA refers to a HA having a molecular weight of about 800-2000, 800-1900, 800-1800, 800-1700, 800-1600, 800-1500, 900-2000, 900-1900, 900-1800, 900-1700, 900-1600, 900-1500, 1,000-2000, 1,000-1900, 1,000-1800, 1,000-1700, 1000-1600 or 1000-1500 KD, in particular, a HA of about 800-1500 KD.

In one embodiment, the kit comprises a glucocorticoid selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof. In one embodiment, the glucocorticoid comprises dexamethasone, budesonide, or a derivative thereof.

In a fifth aspect, provided is use of a small molecular weight HA in the preparation of an agent for increasing the efficacy of a PEG-IL2 and/or a glucocorticoid in treating atopic dermatitis; and a small molecular weight HA for use in increasing the efficacy of a PEG-IL2 and/or a glucocorticoid in treating atopic dermatitis.

The small molecular weight HA refers to a HA having a molecular weight of less than or equal to about 300 KD, such as about 250, 200, 150, 100, 90, 80, 70, 60, 50, 40, 30, 20, 10, 9, 8, 7, 6, 5, 4, 3 or 2 KD. In one embodiment, the small molecular weight HA refers to a HA having a molecular weight of about 2-20, 2-15, 2-10, 2-5, 3-20, 3-15, 3-10 or 3-5 KD, e.g., about 5 KD, 4 KD, 3 KD or 2 KD. In one embodiment, the small molecular weight HA refers to a HA having a molecular weight of about 4 KD.

In one embodiment, provided is use of a large molecular weight HA and a small molecular weight HA in the preparation of an agent for increasing the efficacy of a PEG-IL2 and/or a glucocorticoid in treating atopic dermatitis; and a large molecular weight HA and a small molecular weight HA for use in increasing the efficacy of a PEG-IL2 and/or a glucocorticoid in the treatment of atopic dermatitis, wherein the large molecular weight HA and the small molecular weight HA are at any ratio, provided that the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less, preferably, the mass ratio of the large molecular weight HA to the small molecular weight HA is about 1:2.

The large molecular weight HA refers to a HA having a molecular weight of greater than or equal to about 800 KD, such as greater than or equal to about 900, 1000, 1100, 1200, 1300, 1400, 1500, 1600, 1700, 1800, 1900, 2000 KD or greater. In one embodiment, the large molecular weight HA refers to a HA having a molecular weight of about 800-2000, 800-1900, 800-1800, 800-1700, 800-1600, 800-1500, 900-2000, 900-1900, 900-1800, 900-1700, 900-1600, 900-1500, 1,000-2000, 1,000-1900, 1,000-1800, 1,000-1700, 1000-1600 or 1000-1500 KD, in particular, a HA of about 800-1500 KD.

In one embodiment, the atopic dermatitis is atopic dermatitis with skin lesions. In particular, the agent for treating atopic dermatitis is a local or topical agent.

In a sixth aspect, provided is use of a 10KD PEG modified IL2 in the preparation of an agent for treating a type I allergy. In particular, the agent for treating a type I allergy is a local or topical agent.

In one embodiment, the modified IL2 is modified by a branched or unbranched PEG. In one embodiment, the modified IL2 is modified by a branched PEG. In one embodiment, the modified IL2 is modified by a non-branched PEG.

In one embodiment, the PEG modification is at the N-terminal amino acid residue of IL-2, for example, the lysine, serine, or threonine residue at the N-terminus of IL-2. In one embodiment, the PEG modification is at the N-terminal α-amino group of IL-2. The PEG modification may be, for example, a single-site modification or multi-site modification, preferably a single-site modification.

In one embodiment, the IL-2 is an IL-2 from human or non-human mammals such as, for example, bovine, rat, mouse, dog, monkey, goat, sheep, cow, deer, horse and cat.

In one embodiment, the IL2 comprises the amino acid sequence of SEQ ID NO: 1.

In one embodiment, the Type I allergy comprises 1) systemic anaphylaxis, including drug anaphylactic shock and serum anaphylactic shock, 2) respiratory allergy such as allergic rhinitis and allergic asthma, 3) gastrointestinal allergy such as allergic gastroenteritis, 4) skin allergy such as urticaria, atopic dermatitis (eczema), and angioedema.

In a seventh aspect, provided is a method for treating a type I allergy in a subject, comprising administering to the subject a 10KD PEG modified IL2.

In one embodiment, the method administered a 10KD PEG modified IL2 which is modified with a branched or unbranched PEG of about 10 KD. In one embodiment, the IL-2 is modified with a branched PEG of 10KD. In one embodiment, the IL-2 is modified with an unbranched PEG of 10KD.

In one embodiment, the PEG modification is at the N-terminal amino acid residue of IL-2, for example, the lysine, serine or threonine residue at the N-terminus of IL-2. In one embodiment, the PEG modification is at the N-terminal α-amino group of IL-2. The PEG modification may be, for example, a single-site modification or multi-site modification, preferably a single-site modification.

In one embodiment, the IL-2 is an IL-2 from humans or non-human mammals such as, for example, bovine, rat, mouse, dog, monkey, goat, sheep, cow, deer, horse, cat, and the like.

In one embodiment, the administered IL2 comprises the amino acid sequence of SEQ ID NO: 1.

In one embodiment, the Type I allergy comprises: 1) systemic anaphylaxis, including drug anaphylactic shock and serum anaphylactic shock; 2) respiratory allergies such as allergic rhinitis and allergic asthma; 3) gastrointestinal allergies such as allergic gastroenteritis; 4) skin allergies such as urticaria, atopic dermatitis (eczema), and angioedema.

In one embodiment, the subject is a human subject or non-human subject having atopic dermatitis, non-limiting examples including humans and other mammals such as cows, rats, mice, dogs, monkeys, goats, sheep, cows, deer, horses, cats, and other non-mammals. In some embodiments, the subject is human.

The administration may be preformed via any route suitable for treating allergies, such as intravenous, local, topical, subcutaneous, intraperitoneal, etc. In one embodiment, the administration is local administration, such as topical application.

The agent may be in any suitable dosage form, such as powder, spray, foam, solution, ointment, emulsion, or the like, and/or may be in a solid, liquid, gel or other form. The agent may also comprise an adjunct such as a pharmaceutically acceptable carrier described previously herein.

In an eighth aspect, a 10KD PEG modified IL2 is provided.

In one embodiment, the IL-2 is an IL-2 of human or non-human mammals such as, for example, bovine, rat, mouse, dog, monkey, goat, sheep, cow, deer, horse, cat, and the like. In particular, the IL-2 comprises the amino acid sequence of SEQ ID NO: 1, and preferably, IL-2 is modified by a PEG at a single site at its N-terminus.

The PEG can be a PEG of any suitable type, such as linear or non-linear PEG. "About 10KD" means the sum of the atomic weights of the atoms constituting the PEG is 10 KD ± 10%, 10 KD ± 5%, 10 KD ± 4%, 10 KD ± 3%, 10 KD ± 2%, 10 KD ± 1%, 10 KD ± 0.5%, or even 10 KD ± 0.1%.

As used herein, the word "or" is intended to include "and" unless indicated otherwise in the context.

As used herein, "optional" or "optionally" means that the subsequently described event or circumstance occurs or does not occur, and this description includes instances where the event or circumstance occurs and instances where the event or circumstance does not occur. For example, "optionally a step" means the step is present or not; "optionally a pharmaceutically acceptable carrier" means to include the pharmaceutically acceptable carrier or not.

As used herein, the term "about" refers to a range of values including a particular numerical value, and one skilled in the art can reasonably assume it to approximate the particular numerical value. In certain embodiments, the term "about" refers to within a standard error using measurements generally accepted in the art. For example, in certain embodiments, about refers to ± 10%, ± 5%, ± 4%, ± 3%, ± 2%, ± 1%, or even ± 0.5% of a particular numerical value.

As used herein, when particular numerical values or ratios are listed for a feature in the description, a range of any two numerical values or ratios thereof is also encompassed. For example, when numbers 1, 2, 3 and 4 are listed, 1-2, 1-3, 1-4, 2-3, 2-4 and 3-4 are also covered.

### Examples

The present invention is further illustrated by the following examples, but any example or combination thereof should not be construed as limiting the scope or embodiment of the invention. The scope of the invention is defined by the appended claims, which is apparent to those of ordinary skill in the art in view of the present specification and common knowledges in the art. Without departing from the spirit and scope of the invention, those skilled in the art can make any modifications or changes to the technical solutions of the invention, which modifications and changes also fall into the scope of the invention. Unless otherwise specified, the methods used in the following examples are conventional methods.

### Materials and Methods

### 1. Experimental Materials

### (I) Experimental Instruments and Consumables

| Names | Brand |
|---|---|
| Fluorescent inverted microscope | AMG EVOS |
| Flow Cytometry | BD |
| Centrifuge | Thermo Fisher Scientific |
| Fluorescence Quantitative PCR Instrument | Roche |
| Microplate reader | TECAN |
| Pipette | Eppendorf |
| Vortex oscillator | Kylin-Bell |
| Animal shaver | Panasonic |
| Surgical instruments | Medical Instrument Company |
| Centrifuge tube | Costar |
| Flow Cytometry Tubes | BD |
| 70 µm screen | Biosharp |
| BUXCO Pulmonary Function Test for Small Animals | Buxco Electronics |

### (II) Experimental Materials and Reagents

### 1. Kits

| Name | Manufacturer |
|---|---|
| SuperReal PreMix Plus (SYBR Green) | TIANGEN |
| PrimeScript^{™} RT reagent Kit with gDNA Eraser | TaKaRa |
| Mouse IgE ELISA Kit | NEOBIOSCIENCE |
| True-Nuclear^{™} Transcription Factor Buffer Set | Biolegend |

### 2. Flow Antibodies

| Name | Manufacturer |
|---|---|
| anti-mouse CD16/32 | Biolegend |
| Anti-mouse CD3 (FITC) | Biolegend |
| Anti-mouse CD4 (APC) | Biolegend |
| Anti-mouse CD25 (PE/CY7) | Biolegend |
| Anti-mouse FOXP3(PE) | Biolegend |
| Anti-mouse PD-1 (BV605) | Biolegend |

### 3. Other Agents

| Name | Manufacturer |
|---|---|
| Chicken ovalbumin | Aladdin |
| Hyaluronic Acid (small molecular weight, 4K) | MeilunBio |
| Hyaluronic acid (large molecular weight, 800,000 - 1,500,000, average > 1,000,000) | MeilunBio |
| IL-2 | Gift of Xiamen Amoytop Biotech Co., LTD. |
| Budesonide suspension | Huadong Hospital |
| Dexamethasone Sodium Phosphate Solution | Huadong Hospital |
| PBS (Phosphate Buffered Saline) | MeilunBio |
| HBSS (Hank's Balanced Salt Solution) | MeilunBio |
| Collagenase NB4 Standard Level | MeilunBio |
| DNase I | MeilunBio |
| Red blood cell lysate | Beyotime |
| Cell staining buffer | Biolegend |
| 4% paraformaldehyde fixing solution | Beyotime |
| Pentobarbital Sodium | Merk |
| Trizol | Invitrogen |
| Anhydrous ethanol | Sinopharm Group |
| Chloroform | Sinopharm Group |
| Isopropanol | Sinopharm Group |
| Internal reference primers for mouse GAPDH | BBI |
| Acetylcholine | Sigma |

### 4. Experimental Animals

Female BALB/c mice, 6 weeks old, SPF grade, purchased from Zhejiang Vital River Laboratory Animal Co., Ltd., were raised in the experimental animal department of Fudan University. In this experiment, the animals were fed and treated in accordance with the international criteria for experimental animal use, and all animals started animal experiments one week after quarantine and experimental environment adaptation.

### Example 1: Preparation of Polyethylene Glycol (PEG) Modified Interleukin 2

There were two structure types for PEG having a molecular weight of 10 kDa and 20 kDa: branched (Y-AALD-10kD, Y-AALD-20kD) and unbranched (M-ALD-10kD, M-ALD-20kD), and all PEG having a molecular weight of 40 kDa were unbranched (M-ALD-40kD (all purchased from BEIJING JIANKAI TECHNOLOGY CO., LTD.).

IL-2 (its sequence was set forth in SEQ ID NO: 1) replacement buffer was acetic acid sodium acetate buffer (pH 4-6), mixed with M-ALD-20kD (BEIJING JIANKAI TECHNOLOGY CO., LTD.) unbranched PEG proportionally (mass ratio: 1:2-1:6), reduced using sodium cyanoborohydride, and reacted at 2-10°C for 3-18 hours to obtain a IL-2(PEG) crude product. The IL-2(PEG) crude product was initially isolated by cation exchange chromatography in acetic acid sodium acetate buffer system, and the target protein peak was collected for reverse phase chromatography, eluted with the gradient of acetonitrile trifluoroacetic acid system, the peak of the target protein was collected for cation exchange chromatography and concentrated under the acetic acid sodium acetate buffer system, the target protein peak was collected and finally was replaced to acetic acid sodium acetate buffer by ultrafiltration, and furthermore, IL-2 modified with 10kDa, 20kDa and 40kDa non-branched PEG and IL-2 modified with 10kDa and 20kDa branched PEG were prepared, and the process was consistent with that for preparing IL-2(PEG) modified with 10kDa and 20kDa non-branched PEG.

The expression manner of the obtained interleukin 2 modified with PEG of different molecular weights and structures was 10kD PEG-IL-2, 20kD PEG-IL-2 and 40kD PEG-IL-2 for non-branched, and 20kD(branched)-IL-2, 10kD PEG(branched)-IL-2 for branched, as well as other types were not shown which had the similar electropherogram.

The main modification site of the selected PEG type was the N-terminus of the peptide chain, after modification by 20kD unbranched PEG, the molecular weight of recombinant human IL-2 (normal) increased from 15kD to 35kD, with a higher purity (FIG. 1a, FIG. 1b). The activity was detected by CTLL-2 cell assay, confirming that the PEG-modified IL-2 was comparable to the IL-2 working standard in specific activity (FIG. 1c, FIG. 1d).

The sequence of human IL-2 was shown in SEO ID NO: 1 (5'-APTSSSTKKTQLQLEHLLLDLQMILNGINNYKNPKLTRMLTFKFYMPKKATELKHLQC LEEELKPLEEVLNLAQSKNFHLRPRDLISNINVIVLELKGSETTFMCEYADETATIVEFL NRWITFAQSIISTLT-3').

### Example 2: Establishment of atopic (allergic) dermatitis mouse model

The mouse atopic dermatitis model was generated by the method with minor modifications according to the literature [JIN H, et al. Animal models of atopic dermatitis. J Invest Dermatol, 2009, 129(1): 31-40; KIM W H, et al. Beneficial effects of melittin on ovalbumin-induced atopic dermatitis in mouse. Sci Rep, 2017, 7(1): 17679; WANG G, et al. Repeated epicutaneous exposures to ovalbumin progressively induce atopic dermatitis-like skin lesions in mice. Clin Exp Allergy, 2007, 37(1): 151-61]. Chicken ovalbumin (OVA) sensitized BALB/c mice were common mouse models of type I allergic diseases. 10µg OVA was mixed with 2 mg of aluminum hydroxide in 200µL of phosphate buffer (PBS) solution, intraperitoneally injected to sensitize mice, once every week (at day 0, 7 and 14), each mouse was injected 3 times, 200µL each time. On day 14, mice were anesthetized using pentobarbital intraperitoneal injection. During anesthesia, the back skin hair was removed with electric clipper and depilatory paste, and after clearing, the medical adhesive tape was directly pasted on the back skin for several seconds and then torn off, repeated about 4 times to destroy the stratum corneum on the surface of skin, and then the OVA patch was pasted on the mouse skin to induce atopic (allergic) dermatitis in mice. The OVA patch was prepared by wetting a 1.5×1.5 cm² sterile gauze in a solution of 1 mg/mL of OVA (dissolved in PBS) (approximately 100µL of OVA solution), and was attached using a transparent dressing to the back skin after epilation and replaced daily for 3-7 days to develop dermatitis manifestation, and the status of the back skin of the mice was recorded photographically on day 4~5 after inducing mouse dermatitis (the dermatitis manifestation was most obvious), and the skin inflammation status was scored. Subsequently, the mice were euthanized, and the skin tissue, spleen and blood of the dermatitis parts were taken for further detection.

### Example 3: Establishment of asthma mouse model

The process for the early sensitization in the mouse was the same as that for type I allergic disease mouse model, i.e. 10µg OVA and 2 mg of aluminum hydroxide were mixed and dissolved in 200µL of phosphate buffer (PBS) solution, intraperitoneally injected to sensitize mice once every week (on day 0, 7 and 14), and each mouse was injected 3 times in total, 200 µL each time. On day 14, mice were challenged with 20µg of sterile saline containing 2% OVA in nasal cavity. Later, the therapeutic drugs were administered locally to the airway of the mice through tip of micro-nebulizer.

### Example 4: Evaluation of Mouse Airway Reactivity

Mice were anesthetized with pentobarbital sodium, then for tracheostomy and mechanical ventilation. Changes in lung resistance following nebulization inhalation of 3.125 mg/ml, 6.25 mg/ml and 12.5 mg/ml of methacholine were measured with an invasive BUXCO Pulmonary Function Test system for Small Animals.

### 1 Grouping and Dosing

During the whole experiment, the mice were randomly divided into 28 groups, 6 mice in each group. The administration mode of each treatment group in AD model mice was the aforementioned patch method, the patch was prepared by wetting a 1.5×1.5 cm² sterile gauze in the drug solution (approximately containing 100µL drug solution and/or 50µL hyaluronic acid), the drugs were dissolved in PBS solution, at a concentration of 100,000 IU/ml of normal IL-2 or PEG-IL-2, 20 µg/ml of budesonide, 100 µg/mL of dexamethasone, and 50 mg/mL of hyaluronic acid (HA). Administration was continued for three consecutive days starting at day 4-5 of induced mouse dermatitis, and was changed every day, except that the administration protocol was different among groups, and the rest conditions remained consistent. After 3 days, the mice were euthanized, and the skin tissue, spleen, and blood of the administered site were taken for further detection. A specific grouping scheme was as follows (except that 10kD PEG had two structures, branched and unbranched chains, and in the following groups, if not indicating the molecular weight of the structure, 40kD PEG and 20kD PEG were both unbranched):
1) In order to verify the effectivity of the drugs for treating dermatitis in mice, the grouping was as follows: (1) blank control group; (2) atopic (allergic) dermatitis (AD) model group; (3) PBS treatment group; (4) budesonide (Bude) treatment group (standard control), 20ug/ml budesonide; (5) 40kD PEG-IL-2 treatment group; (6) 40kD PEG-IL-2+budesonide treatment group; (7) 40kD PEG-IL-2+budesonide +small molecular weight HA treatment group; (8) small molecular weight HA treatment group;
2) In order to explore the influence of PEG-IL-2 of different molecular weights and IL-2 modified with PEG of different structures on the treatment effect, the followings were included: (9) 10kD(unbranched)PEG-IL-2+budesonide+small molecular weight HA treatment group; (10) 10kD(branched)PEG-IL-2+budesonide +small molecular weight HA treatment group; (11) 20kD PEG-IL-2+budesonide+ small molecular weight HA treatment group; (12) normal IL-2+budesonide+small molecular weight HA treatment group;
3) In order to explore the effect of different ratios of large and small molecular weight HA on the treatment effect, the followings were included: (13) 10kD PEG-IL-2+ budesonide+HA (large/small molecular weight HAs = 1:2) treatment group; (14) 10kD PEG-IL-2+budesonide+HA (large/small molecular weight HAs = 1:1) treatment group; (15) 10kD PEG-IL-2+budesonide+HA (large/small molecular weight HAs = 2:1) treatment group; (16) 10kD PEG-IL-2+budesonide+ large molecular weight HA treatment group;
4) In order to compare the effect of different forms of glucocorticoids on the therapeutic effect, the followings were included: (17) 10kD PEG-IL-2+ dexamethasone+HA (large/small molecular weight HAs = 1:2) treatment group, wherein the concentration of dexamethasone was 100 µg/ml, 80-100µl (8-10 µg) each time.
5) In order to explore the long-term therapeutic effect of the drugs: the mouse model of the original atopic (allergic) dermatitis (AD) model, after established successfully, dermatitis was evoked with the OVA patch without any treatment in the control group, and the dermatitis was relieved after separated from the antigen, and then detached from the OVA environment for 6 weeks; after 6 weeks, dermatitis was evoked with the OVA patch again, and the mice were euthanized, and the skin, spleen and blood of the original administration site were taken for further detection; after the experimental group was subjected to OVA for inducing dermatitis, the mice were treated with 10kDa PEG-IL-2+budesonide+HA (large/small molecular weight HAs = 1:2) and then detached from the OVA environment for 6 weeks; after 6 weeks, dermatitis was evoked with the OVA patch again, the mice were euthanized, and the skin, spleen and blood of the original administration site were taken for further detection.
6) Other control groups: (18) sensitized mice were first treated with 10kd PEG-IL-2+ budesonide+small molecular weight HA for 3 days and then dermatitis was induced by OVA; (19) the AD model mice were treated with 10kd PEG-IL-2+HA (small molecular weight) treatment group;
7) Asthma model mice: (20) PBS treatment group; (21) 10kd PEG-IL-2 treatment group: dosing regime was 5000IU 10kDa PEG-IL-2 per mouse per time, once daily for three consecutive days; (22) Combined treatment group, once daily for three consecutive days, 10kd PEG-IL-2(5000 IU)+HA(1.25mg) as the treatment group each time; (23) blank control group
8) In order to learn the difference of induction effect of interleukin 2 modified with PEG of different molecular weights on the regulatory T cells (Tregs), five further groups of physiological model mice were injected intraperitoneally with PEG-IL-2 modified with non-branched PEG of different molecular weight sizes and normal IL-2, with an injection amount of 400,000 IU/time/week.

### 2 Skin Inflammation Status Scoring

Skin inflammation status scores assess dermatitis severity clinically. The total score was 0 to 12, evaluated from four aspects, edema, erythema, scaling and erosion, respectively, an individual score was 0-3 (0, no; 1, mild; 2, moderate; 3, severe), and the final score was the sum of each individual term [SCHWARTZ C, et al. Spontaneous atopic dermatitis in mice with a defective skin barrier is independent of ILC2 and mediated by IL-1β. Allergy, 2019, 74(10): 1920-33].

### 3 Tissue Pathological Staining

After the mice were euthanized, the skin on the back of the mice was taken, and fixed with the fixation solution 4% paraformaldehyde at room temperature for more than 24 hours, dehydrated with alcohol, embedded with paraffin, sectioned longitudally, H&E staining after dewaxing, and observed for skin pathological status under optical microscope.

### 4 Assay the ratio of Treg cells in skin and spleen via flow cytometry

1) Sampling: the mice were euthanized, and the spleen and the skin on the experiment site of the back (about 1.5×1.5 cm²) were taken, and respectively soaked in PBS solution;
2) Tissue treatment: the spleen was grinded and passed through a cell strainer of 70 µm pore size to obtain a single cell suspension of spleen; the skin was first cut to 1×1 mm² size, and then added with HBSS solution containing 2mg/mL collagenase and 1mg/mL DAN enzyme, digested at 37°C for about 50 minutes, and hand-shaken well once every 5 minutes during digestion. after digestion, centrifuging transiently and discarding the supernatant, washing with PBS solution twice, grinding and filtering with a 70 µm filter to obtain a single cell suspension of skin tissue;
3) Lysing red blood cells: after the single cell suspensions were transferred to centrifuge tubes respectively, 3-5 mL of red blood cell lysing solution was added into the tube to resuspend the cells for 2 minutes, centrifuged at 500g and 4°C for 5 minutes, and the supernatant was removed, and the cell pellet was washed twice with PBS;
4) Blocking: counting cells under microscope, taking about 5×10⁶ cells, diluting the CD16/32 antibody at a ratio of 1:100, adding the cell suspension, incubating at 4°C for 15 minutes, and washing with PBS twice;
5) Cell surface antigen staining: CD3, CD4, and CD25 antibodies were diluted in a ratio of 1:100, respectively, and the cell suspension was added, incubated at 4°C in the dark for 30 minutes, and washed twice with PBS;
6) fixation: discarding the supernatant, fixed in the fixation solution in the Transcription Factor Fixation/Permeabilization Buffer Set (Biolegend, 424401) at 4°C for 1 hour in the dark;
7) breaking the membrane: directly adding the membrane breaking solution of the same volume as the fixation solution, centrifuged at 500g and 4°C for 5 minutes, and washing with the membrane breaking solution twice;
8) Intracellular antigen staining: diluting FOXP3 antibody with the membrane breaking solution at a ratio of 1:100, adding the cell suspension, incubated at room temperature in the dark for 1 hour, then washing with the membrane once and with PBS once;
9) Assaying: The cells were resuspended in PBS and analyzed by flow cytometry and FlowJo software.

### 5 Detection of Skin Cytokine Expression by Real-Time PCR

1) Sampling: taking the skin on the back of mice, cutting on ice with ophthalmic scissor and then adding 1mL of Trizol reagent, and homogenizing using tissue homogenizer;
2) RNA extraction: adding 200µL chloroform, shaking vigorously for 15 s, standing at room temperature for 15min, centrifuging at 13000 rpm at 4°C for 15 min, taking the supernatant into another RNase free 1.5mL centrifuge tube, adding an equal amount of isopropanol, invertedly mixing well, standing at room temperature for 15min, centrifuging at 12000 rpm at 4°C for 10min, and discarding the supernatant;
3) Precipitation: adding 30µL of pre-cooled 75% ethanol for washing, and discarding the supernatant after centrifugation;
4) dissolving: adding 30µL of DEPC water to dissolve RNA, and measuring RNA concentration by spectrophotometer;
5) Reverse transcription: according to the measured RNA concentration, strictly following the procedure according to the instruction of the SYBR Green Reverse Transcription kit (TIANGEN, FP205-01), and obtaining 1µg of cDNA per tube via the reverse transcription;
6) RT-PCR loading and detection: selecting 20µL reaction system, operating strictly according to the instruction of the TR-PCR kit (Takara, RR047A), loading on the PCR instrument and analyzing the data using Excel software;
7) The primers in the experiment were as follows:

**Table 1: Primer Sequence**

| **Gene** | **Forward primer (5' to 3')** | **Reverse primer (5' to 3')** |
|---|---|---|
| **GAPDH** | Purchased from BBI, B661304-0001 | Purchased from BBI, B661304-0001 |
| **INF-y** | TATCTGGAGGAACTGGCAAA (SEQ ID NO: 2) | ATCAGGTGTGATTCAATGACG (SEQ ID NO: 3) |
| **IL-4** | TCACTGACGGCACAGAGCTA (SEQ ID NO: 4) | CCTTCTCCTGTGACCTCGTT (SEQ ID NO: 5) |
| **IL-13** | CCTGGCTCTTGCTTGCCTT (SEQ ID NO: 6) | GGTCTTGTGTGATGTTGCTCA (SEQ ID NO: 7) |
| **IL-17** | TGGGTTCTGCGGTATTTGAC (SEQ ID NO: 8) | GCTGCTGTGTGAGGTTGAGC (SEQ ID NO: 9) |

### 6 Assay Mouse Serum IgE Levels by ELISA

1) Sampling: the mice were anesthetized with pentobarbital, and the heart blood was collected for about 500 mL and allowed to stand at room temperature for 1 hour. After blood collection, the mice were euthanized. After one hour, centrifuging at 3500 rpm at room temperature for 15 min to obtain the serum;
2) Referring to the pre-experimental results, after the serum was diluted at a factor of 4 or 10, it was strictly operated according to the instruction of the mouse serum IgE ELISA kit (NEOBIOSCIENCE, EMC117.48), and the standard sample and serum samples were simultaneously assayed, a duplicate well was set for each well, the development result was measured using a microplate reader at the detection wavelength of 450 nm.
3) The serum IgE concentration was calculated according to the standard curve and the curve equation obtained by mapping using Excel.

### III. Data Analysis and Processing Method

Flowjo software collates streaming data. The Data were analyzed statistically using Graphpad Prism 7 software or Excel software, and the results were expressed by mean ± standard error, where P value < 0.05 (* indicates) that there is a difference between data, and the more the symbol *, the smaller the value of P, and the more significant the difference between data.

### Results

### 1. OVA Induces Mouse Skin Inflammation

The study used OVA to induce a mouse atopic dermatitis (AD) model, as specifically described previously. Compared with the blank control, the back skin of mice in the AD model group showed obvious edema, erythema, scaling, erosion, etc. (FIG. 2A), and the score of the skin state also increased from 0 to an average of 6.99 (Table 2). The pathological section results showed that dermatitis mice had thickened skin, incomplete stratum corneum, and large inflammatory cells infiltration (FIG. 2B). We used RT-PCR method to detect the change in the levels of inflammatory factors on the back skin, and the results showed that compared with the blank control mice, the expressions of Th2 cytokines IL-4 and IL-13 in skin of AD mice were increased and the expression of Th17 cytokine IL-17 was also increased, while the expression of Th1 cytokine IFN-γ did not change significantly (FIG. 2C), the concentration of IgE in serum of AD mice detected by ELISA method was also significantly increased (FIG. 2D), and the allergic disease model of BALB/c mice induced by OVA was a classical Type I allergy disease model.

**Table 2: mice dermatitis scoring table ( χ̅±s): 6 mice per group, scored respectively from four aspects, edema, erythema, scale and erosion, and the scores were summed. The total score was 0-12. Using the PBS treatment group as the control, * indicates P < 0.05, ▲ indicates P < 0.01. ▲ ▲ represents P > 0.05 when the normal IL-2+budesonide+small molecular weight HA group was compared to the budesonide group.**

| **Grouping** | **n** | **Dermatitis score (**χ̅±**s**) |
|---|---|---|
| **Blank** | 6 | 0±0 |
| **Inflammatory status** | 6 | 6.99±1.414^{▲} |
| **PBS Treatment** | 6 | 4.333 ±1.506 |
| **40kd PEG-IL-2 alone** | 6 | 2.667±0.516 |
| **10kd PEG-IL-2 alone** | 6 | 2.833±0.753 |
| **10kd PEG-IL-2+small molecular weight HA** | 6 | 2.5±1.049* |
| **Budesonide alone** | 6 | 2.333±1.211* |
| **40KD PEG-IL-2+Bude** | 6 | 3.16711.169 |
| **small molecular weight HA alone** | 6 | 3.167±0.753 |
| **The normal skin of sensitized mice was first administered with 10kd PEG-IL-2+Bude+small** | 6 | 6.167±1.169* |
| **molecular weight HA, and after 3 days, induced by contacting OVA.** | | |
| **40KD(unbranched)PEG-IL-2+Bude+small molecular weight HA** | 6 | 1.167±0.753^{▲} |
| **20KD(unbranched)PEG-IL-2+Bude+small molecular weight HA** | 6 | 1.333±0.516^{▲} |
| **10KD branched PEG-IL-2+Bude+small molecular weight HA** | 6 | 1.500±0.547^{▲} |
| **10KD(unbranched) PEG-IL-2+Bude+small molecular weight HA** | 6 | 1.167±0.408^{▲} |
| **normal IL-2+Bude+small molecular weight HA** | 6 | 2.333±0.516*^{▲▲} |
| **10KD PEG-IL-2+Bude+1/3 large molecular weight HA+2/3 small molecular weight HA** | 6 | 1.000±0.633^{▲} |
| **10KD PEG-IL-2+Bude+1/2 large molecular weight HA+1/2 small molecular weight HA** | 6 | 1.500±0.836^{▲} |
| **10KD PEG-IL-2+Bude+2/3 large molecular weight HA+1/3 small molecular weight HA** | 6 | 1.000±0.633^{▲} |
| **10KD PEG-IL-2+Bude+ large molecular weight HA** | 6 | 2.000±0.633* |
| **10KD PEG-IL-2+Dexamethasone+1/3 large molecular weight HA+2/3 small molecular weight HA** | 6 | 1.500±0.547^{▲} |
| **(10KD PEG-IL-2+Bude+1/3 large molecular weight HA+2/3 small molecular weight HA) 6 weeks after treatment** | 6 | 2.167±0.753* |

**2. PEG-IL-2 in combination with budesonide has limited therapeutic effect on atopic dermatitis model mice.**

In the previous study, our team had promoted Treg cell proliferation and upregulated the odds ratio of Treg/Tcon cells in lung alveolar lavage fluid in asthma model mice treated with 40kDa polyethylene glycol modified IL-2 (PEG-IL-2) in combination with dexamethasone via short-term nebulization inhalation. It is possible to significantly reduce airway hypersensitivity responsiveness, correct abnormal polarization of immune Th2 cells [WU K, et al. Short-term intratracheal use of PEG-modified IL-2 and glucocorticoid persistently alleviates asthma in a mouse model. Sci Rep, 2016, 6(31562]. We therefore attempted to treat mouse dermatitis by topical skin administration using the same protocol, with the model group and the PBS treatment group as controls. After 3 days of administration, compared with AD model mice, it was observed that the dermatitis of mice in each of the treatment groups were relieved to different extents, and the dermatitis scores all were decreased. Compared with the PBS treatment group, the dermatitis score of the budesonide alone group was decreased more and there was statistical difference (P < 0.05), but there was no statistical difference in the dermatitis scores of the 40kD PEG-IL-2 alone group and the 40kD PEG-IL-2+budesonide group compared to the PBS group (Table 2, FIG. 3C). The pathological section showed no significant improvement in the skin thickness of the 40kD PEG-IL-2 alone group and the PBS group as well as the infiltration of inflammatory cells, and there was no significant change in the proportion of CD4+CD25+FOXP3+ regulatory T cells (Tregs) in skin CD4+ cells in the skin of mice detected by flow cytometry. However, the skin thickness of mice in the groups treated with budesonide alone (currently clinically standard first-line treatment regimen) and with 40kD PEG-IL-2 combined with budesonide was relatively reduced, the infiltration of inflammatory cells was relatively reduced, the increase of Treg cell proportion in skin was statistically different compared with that of the physiological model control group (blank control), but there was no statistical difference in the proportion of Tregs among the treatment groups (FIG. 3).

**3. HA can enhance the therapeutic effects of PEG-IL-2 combined budesonide on atopic dermatitis in mice.**

The above results showed that the original drug combination regimen (i.e. 40kD PEG-IL-2 combined glucocorticoid) had no improvement in efficacy compared with the clinical standard protocol (glucocorticoid), and we assumed it is because the drugs do not enter into the deep layer of epidermis or dermis layers to function. In order to promote drug absorption, another group of dermatitis mice were further administered with a small molecular weight HA on the basis of the original drug combination regimen, namely 40kD PEG-IL-2+budesonide+small molecular weight HA, for 3 days. The results showed that, compared with the group without HA, skin inflammation in mice of the group further treated with HA showed obvious and rapid alleviation, skin was recovered to be normal, the dermatitis score was decreased and there was statistical difference (Table 2, FIG. 4D), the pathological section showed a significant reduction in skin thickness, there was significant reduction in inflammatory cell infiltration, and the proportion of Treg in skin detected by flow cytometry was increased to 19.8%, and the difference was statistically significant. In addition, although the control group with the small molecular weight HA alone can relieve the symptoms of dermatitis to some extent, but had no upregulation effect on skin Treg (FIG. 4), which may be related to the moisturizing effect of HA molecules, and also proved that the small molecule HA has no immunomodulatory effect.

**4. HA adjuvant combined drug treatment on dermatitis mice can prevent relapse of dermatitis again, and application on normal skin of only sensitized mice had no such preventive effect.**

As described above, after first OVA-inducing dermatitis, we administered HA+PEG-IL-2+budesonide in combination for 3 days, and attemped to evoke dermatitis by topical external application of OVA again, and mice treated with PBS for 3 days were used as controls. The results showed no obvious dermatitis manifestation occurred in mice of the treatment group after 7 days of topical external OVA application, while the mice in the control group showed dermatitis manifestation, and the severity was comparable to the first induction. At the same time, we selected another group of mice that had been intraperitoneally injected with OVA for sensitization but yet no topical OVA-induced dermatitis, firstly, PEG-IL-2+budesonide+small molecular weight HA was topically applied for 3 consecutive days, and dermatitis was induced against by OVA patch. The results showed that dermatitis manifestation was induced successfully by OVA patch for about 2-4 days, and there was no statistical difference in dermatitis scores and the ratio of skin Treg cells compared to AD model mice (FIG. 5); if no dermatitis (no skin lesion) was induced after antigen-sensitization, HA may not help the drugs to permeate the skin.

### 5. Effect of IL-2 Modified with PEG of Different Molecular Weights and Different Structures in the drug combination on Treating AD Mice

To explore whether the molecular weight size of PEG-IL-2 and the modification mode of PEG to IL-2 play a key role in the therapeutic effect of the drug combination, we further included four treatment groups, 20kD PEG-IL-2+budesonide+small molecular weight HA, 10kD branched PEG-IL-2+budesonide+small molecular weight HA, 10kD (unbranched) PEG-IL-2+budesonide+small molecular weight HA and normal IL-2+budesonide+small molecular weight HA, and used the treatment group of 40kD PEG-IL-2+budesonide+small molecular weight HA described above as a control (the 40kD PEG and 20kD PEG without specifying the structure were unbranched). The results showed that application of the treatment groups of PEG-IL-2 of different molecular weight sizes and modification modes can obviously alleviate the symptoms of mouse dermatitis, the dermatitis score was obviously decreased and the inter-group difference was not statistically significant (see Table 2 and FIG. 6D), but as the molecular weight of the PEG-IL-2 became smaller, the ratio of Treg cells in skin became higher, and the inter-group difference was statistically significant; in the case of 10kDa PEG molecules, there was no statistical difference in the Treg ratios between the unbranched PEG-IL-2 group and the branched PEG-IL-2 group. Interestingly, the molecular weight of normal IL-2 (15 kDa) is lower than that of PEG-IL-2 (40/20/10+15 kDa), but the therapeutic effect of the normal IL-2+budesonide+small molecular weight HA treatment group was significantly weaker than that of each of the PEG-IL-2+budesonide+small molecular weight HA treatment groups, the skin still had a few scaling phenomena; compared with the 40kD PEG-IL-2+budesonide+small molecular weight HA treatment group, the dermatitis scores were higher, with an average of 2.33, and there was statistical difference (P < 0.05), and the Treg ratio in the skin was also the lowest, with a statistical difference (P < 0.05) (FIG. 6). In dermatitis score of the normal IL-2+ budesonide+small molecular weight HA group, there was no difference (p < 0.05) compard to the budesonide alone group (P > 0.05); there was no difference between these two groups in inducing FOXP3⁺CD25⁺ T cells in skin (p > 0.05), indicating that the normal interleukin 2 had no work (see Table 2, FIGs. 6D and E). The small molecular weight HA did not help the normal IL-2 having a molecular weight of 15kDa to enter into the deep layer of the skin through the epidermis layer, while can help the 10kD PEG-IL-2 (25kDa), 20kD PEG-IL-2 (35kDa) and 40kD PEG-IL-2 (55kDa) having a molecular weight of substantially greater than 15kDa, and modified hydrophilic polar PEG to enter the deep layer of the skin to exert therapeutic effects. We thus unexpectedly found that HA can allow an extra-large molecular weight protein drug to enter through the stratum corneum and the surface of the skin by interacting with PEG, or remain in dermis layer (atopic dermatitis immunoreactive layer).

### 6. 10kd-PEG-IL-2 promotes regulatory T cell (Treg) to highly express FoxP3 and PD-1

We intraperitoneally injected PEG-IL-2 modified with non-branched PEG of different molecular weight sizes in physiological model mice, and detected FoxP3+ regulatory T cells and the expression of PD-1 in spleen cells on day 4. We unexpectedly found that 10kd-PEG-IL-2 up-regulated Treg cells, while the normal IL-2, 20kd-PEG-IL-2 and 40kd-PEG-IL-2 did not up-regulate Tregs. The level of the regulatory T cells in the 10kd-PEG-IL-2 group was higher than that of the normal IL-2 group, the 20kd-PEG-IL-2 group and the 40kd-PEG-IL-2 group, and the difference was statistically significant (see FIG. 7A).

In addition, we examined the expression level of PD-1 on the surface of Treg, and found that normal IL-2 can promote the expression of PD-1 by regulatory T cells. The expression level of PD-1 on Treg in the 10kd-PEG-IL-2 group was also higher than that of the PBS group (18.1% vs 13.1%), but the difference was not statistically significant. The expression level of PD-1 on the surface of Treg in the 10kd-PEG-IL-2 group was similar to that of the normal IL-2 group, and the difference was not statistically significant (FIG. 7B). Treg cells express PD-1, enhancing the inhibitory effect of Treg cells on effector T cells (Teff).

**7. 10kd PEG-IL-2 alone can up-regulate the proportion of Treg cells in skin and have a therapeutic effect on atopic dermatitis.**

We treated dermatitis in AD mice by topical external application of 10kd PEG-IL-2 and 10kd PEG-IL-2+HA, and after 3 days, it was found that the symptoms such as swelling and erosion of the skin in the two groups of mice were relieved, but the skin was still rough, the rash was not completely retreated, the score of dermatitis was decreased, with statistically significane compared with the original AD model mouse; when compared between the two groups, the mouse skin with 10kd PEG-IL-2+HA was smoother and the dermatitis score was lower. The pathological section also showed that the thickened status in the two groups was relieved, and inflammatory cell infiltration was reduced. The results showed that compared with the AD model mice, CD4+CD25+FoxP3+ Treg cells in the skin of the two treatment groups were up-regulated, and the difference was statistically significant, but there was no statistical difference between the two groups (FIG. 8).

**8. 10kd PEG-IL-2 can relieve airway hyperresponsiveness in asthma mice.**

4 groups of mice were further included, and the 10kd PEG-IL-2 was further investigated for its immunomodulatory action in another classical allergy model, mouse asthma model. After establishing the mouse asthma model successfully, the mice were divided into 3 groups, and respectively sprayed through mouse trachea with PBS, 10kd PEG-IL-2 and 10kd PEG-IL-2+HA via micronebulizer, and after 3 days, the airway responsiveness of the mice was evaluated using healthy mice as blank control.

As shown in FIG. 7, the results showed that the 10kd PEG-IL-2 effectively reduced airway resistance change of asthma mice, reduced airway hyperresponsiveness, and had a statistical difference compared with the blank control group and the PBS group. However, in the asthma mouse model, HA cannot further improve the effect of relieving the high-resistance state of the airway by the 10kd PEG-IL-2.

### 9. Effect of Different Ratios of Large/Small Molecular Weight HA in the combination on the treatment of AD Mice

As described above, small molecular weight HA had a strong auxiliary drug absorption effect without an immunomodulatory function, and large molecular weight HA has a relatively poor skin permeability but can enhance the immunomodulatory capability of activated Treg cells in local environment. Therefore, after the immunomodulatory effect of the 10kDa PEG-IL-2 was determined, in order to further explore the influence of the large/small molecular weight HA and their ratio in the combination on the treatment of AD mice, we additionally selected 4 groups of AD model mice, and keeping the 10kDa PEG-IL-2+budesonide unchanged, further combined the large molecular weight HA, 2/3 large molecular weight HA+1/3 small molecular weight HA, 1/2 large molecular weight HA+1/2 small molecular weight HA, 1/3 large molecular weight HA+2/3 small molecular weight HA, respectively, taking the aforementioned combination with small molecular weight HA as a control. The results showed that each group obviously and quickly relieved the symptoms of dermatitis, and the dermatitis score was reduced, but for the 1/2 large molecular weight HA+1/2 small molecular weight HA group and the large molecular weight HA group, when respectively compared with the budesonide alone group, the improvement of inflammation was not significant (Table 2, FIG. 10D). The pathological section showed that the skin thickness of mice in each group was obviously thinned, the infiltration of inflammatory cells was significantly reduced, and the small molecular weight HA group and 1/3 large molecular weight HA+2/3 small molecular weight HA group were the best, and the 1/3 small molecular weight HA+2/3 large molecular weight HA group was the second best. The flow cytometry detection of the skin of the mice showed that, except the 1/2 large molecular weight HA+1/2 small molecular weight HA group and the large molecular weight HA group were not statistically different from the budesonide alone group, other groups were able to up-regulate the ratio of skin Treg, in which the small molecular weight HA group and the 1/3 large molecular weight HA+2/3 small molecular weight HA group resulted in the highest ratio of upregulation and were significantly different from the budesonide group. (FIGS. 10C, E). The above indicates that both the large molecular weight HA and the small molecular weight HA can enhance the treatment of a PEG-IL-2 combined budesonide on mice dermatitis, but it is favored that the effect of small molecular weight HA is greater. The small molecular weight hyaluronic acid can help the PEGylated protein drug interleukin 2 penetrate and remain in the skin immunoreaction layer, the interaction between the small molecular weight hyaluronic acid and the PEG is a requisite for entry of the large interleukin 2 protein drug into skin, and skin lesion can destroy the structure of the stratum corneum of skin such as keratinized membrane and lipid membrane and stratum granulosum, the final defense-line of skin barrier, and it has not been determined whether it is a requisite to help interleukin 2 enter into skin to exert immunological functions. For hydrophilic large molecular weight HA, considering the immunomodulatory function of the large molecular weight HA, it is believed that the administration protocol of the 10kDa PEG-IL-2+budesonide+1/3 large molecular weight HA+2/3 small molecular weight HA can not only up-regulate the ratio of Treg in skin, but also enhance the function of Treg, thus it is the current optimal dosage regimen.

**10. The type of glucocorticoid has no significant effect on the efficacy of the drug combination.**

To explore the effects of different types of glucocorticoids in combination PEG-IL-2 and HA on the treatment of dermatitis in mice, another group of mice was taken and administered with a combination of 10kDa PEG-IL-2+dexamethasone (+HA (large : small was 1:2), using 10kDa PEG-IL-2+budesonide+HA (large : small was 1:2) as a control. The results showed that both groups obviously and quickly relieved the symptoms of dermatitis, reduced infiltration of inflammatory cells, up-regulated the ratio of Treg in skin, and there was no statistically significant difference between the two groups of data (see Table 2 and FIG. 11).

**11. The administration of 10kDa PEG-IL-2 combined budesonide and 1/3 large molecular weight HA+2/3 small molecular weight HA is significantly superior to the clinical first-line protocol (topical external application of glucocorticoid) in efficacy.**

As described above, the administration protocol of 10kDa PEG-IL-2 combined budesonide and 1/3 large molecular weight HA+2/3 small molecular weight HA was selected as the best solution, which was further compared with the current clinical first-line treatment protocol (local external application of glucocorticoid, that is, the budesonide alone group), we detected the level change of skin inflammatory factors IL-4, IL-13, IL-17 and IFN-γ in the two groups of mice using RT-PCR, and detected the serum IgE concentrations of the two groups of mice using ELISA method, using the blank group and the AD model group mice as controls. The results showed that compared with the control group, the expressions of skin IL-4, IL-13 and IL-17 of the mice in the two treatment groups were downregulated, in which the drug combination group was more obvious, while there was no difference in IFN-γ expression (P > 0.05). The serum IgE concentrations of the two groups were also decreased, in which that of the drug combination group decreased more (see FIGS. 12A, 12B). All the above data demonstrated that the drug combination regimen selected by us (10kDa PEG-IL-2 combined budesonide and 1/3 large molecular weight HA+2/3 small molecular weight HA) has a significantly better therapeutic effect than the clinical first-line treatment protocol (the budesonide alone group).

### 12. The efficacy of the regimen of 10kDa PEG-IL-2 combined budesonide and 1/3 large molecular weight HA+2/3 small molecular weight HA for treating atopic dermatitis in mice lasted for at least 6 weeks.

In order to explore whether the efficacy of the drug combination regimen of 10kDa PEG-IL-2 combined budesonide and 1/3 large molecular weight HA+2/3 small molecular weight HA can last for a long time, we randomly selected two groups of AD model mice, wherein one was treated transiently for 3 days with this regimen, which group was sensitized with OVA and then evoked with OVA patch to induce atopic dermatitis prior to the treatment, and the other group of mice was not treated at all, and after 3 days, both groups of mice were not treated any more and fed under the same conditions. After 6 weeks, the OVA patches were directly pasted on the skin of the two groups of mice to attempt to induce atopic dermatitis in mice, using the untreated group of AD mice as the control group.

The results showed that after 6W, in the control group of AD mice, dermatitis still was induced again, the dermatitis performance severity and the skin Treg ratio were the same as that of the first evoked dermatitis, and it was demonstrated that the mice were still in the OVA sensitization state after 6 weeks, excluding the case that the mice were no longer sensitive to the OVA after 6 weeks. After 6 weeks, in the drug combination group of mice administered with 10kDa PEG-IL-2 combined budesonide and 1/3 large molecular weight HA+2/3 small molecular weight HA, no obvious atopic dermatitis was induced again, the skin remained in normal state, and the dermatitis score was low (see Table 2, FIG. 13A), the pathological section showed the skin was thickened and a large amount of inflammatory cells infiltrated in the AD control group, while in the drug combination group, there was no obvious thickening, and only small number of inflammatory cells infiltrated (FIG. 13B). The ratio of skin Treg was still up to about 19.5% (vs the control group of 14.2%), and the difference was statistically significant (FIGs. 13C, 13D, 13E). The above demonstrated that the dosing regimen of 10kDa PEG-IL-2 combined budesonide and 1/3 large molecular weight HA+2/3 small molecular weight HA can not only effectively relieve the symptoms of atopic dermatitis in mice, but also prevent relapse of dermatitis again, and the effect can last for at least 6 weeks.

## Claims

1. A pharmaceutical composition for treating atopic dermatitis, comprising a polyethylene glycol-modified interleukin 2 (PEG-IL2), a glucocorticoid and a small molecular weight hyaluronic acid (HA), and optionally a pharmaceutically acceptable carrier, wherein the molecular weight of said small molecular weight HA is less than or equal to about 300 KD, preferably is about 2-20KD, more preferably is about 4 KD.

2. The pharmaceutical composition of claim 1, wherein the PEG-IL2 is modified by a polyethylene glycol (PEG) having a molecular weight of less than or equal to about 100, 90, 80, 70, 60, 50 or 40 KD,
preferably, the PEG is a PEG having a molecular weight of about 5-100, 5-90, 5-80, 5-70, 5-60, 5-50 or 10-40 KD,
preferably, the IL-2 comprises the amino acid sequence of SEQ ID NO: 1, more preferably, the IL-2 is modified at its N-terminus at a single site by the PEG.

3. The pharmaceutical composition of claim 1 or 2, wherein the glucocorticoid is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof, preferably selected from dexamethasone, budesonide and derivatives thereof.

4. The pharmaceutical composition of any one of claims 1-3, further comprising a large molecular weight HA, wherein the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less, more preferably, the mass ratio of the large molecular weight HA to the small molecular weight HA is less than or equal to about 1:2, preferably is about 1:2, wherein the molecular weight of the large molecular weight HA is greater than or equal to about 800 KD, for example, is about 800-2000 KD, preferably is about 800-1500 KD.

5. The pharmaceutical composition of any one of claims 1-4, comprising a 10KD PEG modified IL2, a glucocorticoid and a HA, wherein the HA comprises (i) a small molecular weight HA or (ii) a large molecular weight HA and a small molecular weight HA at a mass ratio of about 1:2, preferably the glucocorticoid is selected from the group consisting of dexamethasone, budesonide and derivatives thereof.

6. Use of a PEG-IL2, a glucocorticoid and a HA in the preparation of a medicament or a kit for treatmenting atopic dermatitis, wherein the HA comprises a small molecular weight HA, wherein the molecular weight of the small molecular weight HA is less than or equal to about 300 KD, preferably is about 2-20KD, more preferably is about 4 KD.

7. The use of claim 6, wherein the PEG-IL2 is modified by a polyethylene glycol (PEG) having a molecular weight of less than or equal to about 100, 90, 80, 70, 60, 50 or 40 KD, preferably, the PEG is a PEG having a molecular weight of about 5-100, 5-90, 5-80, 5-70, 5-60, 5-50 or 10-40 KD,
preferably, the IL-2 comprises the amino acid sequence of SEQ ID NO: 1, more preferably, the IL-2 is modified at its N-terminus at a single site by the PEG.

8. The use of claims 6 or 7, wherein the glucocorticoid is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof, preferably selected from dexamethasone, budesonide and derivatives thereof.

9. The use of any one of claims 6-8, wherein the HA further comprises a large molecular weight HA, wherein the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less, more preferably, the mass ratio of the large molecular weight HA to the small molecular weight HA is less than or equal to about 1:2, preferably is about 1:2, wherein the molecular weight of the large molecular weight HA is greater than or equal to about 800 KD, for example is about 800-2000 KD, preferably is about 800-1500 KD.

10. The use of any one of claims 6-9, wherein the PEG-IL2 is a 10KD PEG modified IL2, and the HA comprises (i) a small molecular weight HA; or (ii) a large molecular weight HA and a small molecular weight HA at a mass ratio of about 1:2, preferably, the glucocorticoid is selected from dexamethasone, budesonide and derivatives thereof.

11. A method for treating atopic dermatitis in a subject, comprising administering to the subject a therapeutically effective amount of a PEG-IL2, a glucocorticoid and a small molecular weight HA, wherein the molecular weight of the small molecular weight HA is less than or equal to about 300 KD, preferably is about 2-20 KD, more preferably is about 4 KD.

12. The method of claim 11, wherein the PEG-IL2 is modified by a polyethylene glycol (PEG) having a molecular weight of less than or equal to about 100, 90, 80, 70, 60, 50 or 40 KD, preferably, the PEG is a PEG having a molecular weight of about 5-100, 5-90, 5-80, 5-70, 5-60, 5-50 or 10-40 KD,
preferably, the IL-2 comprises the amino acid sequence of SEQ ID NO: 1, more preferably, the IL-2 is modified at its N-terminus at a single site by the PEG.

13. The method of claim 11 or 12, wherein the glucocorticoid is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof, preferably selected from dexamethasone, budesonide and derivatives thereof.

14. The method of any one of claims 11-13, further comprising administering a large molecular weight HA, wherein the mass ratio of the administered large molecular weight HA to the small molecular weight HA is about 2:1, or the ratio of the mass of the administered large molecular weight HA to the total mass of the large molecular weight HA and the small molecular weight HA is less than 50%, preferably less than 40%, 35%, 30%, 25%, 20%, 15%, 10% or less, more preferably, the mass ratio of the administered large molecular weight HA to the small molecular weight HA is less than or equal to about 1:2, preferably is about 1:2, wherein the molecular weight of the large molecular weight HA is greater than or equal to about 800 KD, such as is about 800-2000 KD, preferably is about 800-1500 KD.

15. The method of any one of claims 11-14, wherein, the administered PEG-IL2 is a 10KD PEG modified IL2, and the administered HA comprises (i) a small molecular weight HA or (ii) a large molecular weight HA and a small molecular weight HA at a mass ratio of about 1:2, preferably, the administered glucocorticoid is selected from dexamethasone, budesonide and derivatives thereof.

16. A kit, preferably for the treatment of atopic dermatitis, comprising a PEG-IL2, a glucocorticoid and a small molecular weight HA, wherein the molecular weight of the small molecular weight HA is less than or equal to about 300 KD, preferably is about 2-20 KD, more preferably is about 4 KD.

17. The kit of claim 16, wherein the PEG-IL2 is modified by a polyethylene glycol (PEG) having a molecular weight of less than or equal to about 100, 90, 80, 70, 60, 50 or 40 KD, preferably, the PEG is a PEG having a molecular weight of about 5-100, 5-90, 5-80, 5-70, 5-60, 5-50 or 10-40 KD,
preferably, the IL-2 comprises the amino acid sequence of SEQ ID NO: 1, more preferably, the IL-2 is modified at its N-terminus at a single site by the PEG.

18. The kit of claim 16 or 17, wherein the glucocorticoid is selected from the group consisting of dexamethasone, budesonide, beclomethasone dipropionate, ciclesonide, hydrocortisone, cortisone, prednisone, prednisolone, methylprednisolone, triamcinolone, betamethasone, clobetasone butyrate, triamcinolone acetonide, fluocinolone, mometasone furoate, halcinonide, clobetasol propionate, halcinonide, halometasone, diflorasone diacetate, mometasone, loteprednol, etiprednol, triamcinolone, flunisolide, flumoxonide, rofleponide, butixocort, tipredane and derivatives thereof, preferably selected from dexamethasone, budesonide and derivatives thereof.

19. The kit of any one of claims 16-18, further comprising a large molecular weight HA, wherein the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less, more preferably, the mass ratio of the large molecular weight HA to the small molecular weight HA is less than or equal to about 1:2, preferably is about 1:2, wherein the molecular weight of the large molecular weight HA is greater than or equal to about 800 KD, for example, is about 800-2000 KD, preferably is about 800-1500 KD.

20. The kit of any one of claims 16-19, comprising a 10KD PEG modified IL2, a glucocorticoid and a HA, wherein the HA comprises (i) a small molecular weight HA or (ii) a large molecular weight HA and a small molecular weight HA at a mass ratio of about 1:2, preferably the glucocorticoid is selected from dexamethasone, budesonide and derivatives thereof.

21. Use of a small molecular weight HA in the preparation of an agent for increasing the efficacy of a PEG-IL2 and/or a glucocorticoid in treating atopic dermatitis, wherein the molecular weight of said small molecular weight HA is less than or equal to about 300 KD, for example is about 2-20 KD, preferably is about 4 KD.

22. Use of a large molecular weight HA and a small molecular weight HA in the preparation of an agent for increasing the efficacy of a PEG-IL2 and/or a glucocorticoid in treating atopic dermatitis, wherein the mass ratio of the large molecular weight HA to the small molecular weight HA is about 2:1, or the ratio of the mass of the large molecular weight HA to the total mass of the large molecular weight HA and the small molecular weight HA is less than 50%, preferably less than 40%, 35%, 30%, 25%, 20%, 15%, 10% or less, more preferably, the mass ratio of the large molecular weight HA to the small molecular weight HA is less than or equal to about 1:2, preferably is about 1:2,
wherein, the molecular weight of the large molecular weight HA is greater than or equal to about 800 KD, such as is about 800-2000 KD, preferably is about 800-1500 KD, preferably, the molecular weight of the small molecular weight HA is less than or equal to about 300 KD, for example is about 2-20 KD, preferably is about 4 KD.

23. Use of a PEG-IL2 in the preparation of a medicament for treating a Type I allergy disease in a subject, wherein the PEG-IL2 is a 10KD PEG modified IL2, preferably, the IL-2 comprises the amino acid sequence of SEQ ID NO: 1, more preferably, the IL-2 is modified at its N-terminus at a single site by the PEG, preferably, the Type I allergy disease is selected from the group consisting of systemic anaphylaxis including drug anaphylactic shock and serum anaphylactic shock, respiratory allergies such as allergic rhinitis and allergic asthma, gastrointestinal allergic reactions such as allergic gastroenteritis, skin allergic reactions such as urticaria, atopic dermatitis (eczema) and angioedema.

24. A method of treating a Type I allergic reaction in a subject, comprising administering to the subject a 10KD PEG modified IL2, preferably, the IL-2 comprises the amino acid sequence of SEQ ID NO: 1, more preferably, the IL-2 is modified at its N-terminus at a single site by the PEG, preferably, the Type I allergy disease is selected from the group consisting of systemic anaphylaxis including drug anaphylactic shock and serum anaphylactic shock, respiratory allergies such as allergic rhinitis and allergic asthma, gastrointestinal allergic reactions such as allergic gastroenteritis, skin allergic reactions such as urticaria, atopic dermatitis (eczema) and angioedema.

25. A 10KD PEG modified IL2, preferably, said IL-2 comprises the amino acid sequence of SEQ ID NO: 1, more preferably, the IL-2 is modified at its N-terminus at a single site by the PEG.
